# EUROPEAN PATENT APPLICATION

(11) **EP 4 349 350 A1**
(43) Date of publication of application: **10.04.2024**
(21) Application number: 21935727.4
(22) Date of filing: 15.07.2021
(51) Int. Cl.: A61K 35/741, A61K 39/02, A61P 1/16, A61P 3/10, A61P 1/04, A61P 9/00, C12N 1/20, C12R 1/01

(54) **BACTERIAL STRAIN, COMPOSITION, DRUG FOR USE IN COMBINATION, AND USE**

(30) Priority: 06.04.2021 CN 202110369840; 06.04.2021 CN 202110370249
(71) Applicant: Moon (Guangzhou) Biotech Co., Ltd., Guangzhou, Guangdong 510535 (CN)
(72) Inventor: LIN, Quansheng, Guangzhou, Guangdong 510535 (CN); JIANG, Xianzhi, Guangzhou, Guangdong 510535 (CN); XIAN, Yibo, Guangzhou, Guangdong 510535 (CN); KUANG, Zupeng, Guangzhou, Guangdong 510535 (CN); HUANG, Baojia, Guangzhou, Guangdong 510535 (CN); KONG, Ping, Guangzhou, Guangdong 510535 (CN); DENG, Qianying, Guangzhou, Guangdong 510535 (CN); ZHAO, Yingying, Guangzhou, Guangdong 510535 (CN); XIAO, Chen, Guangzhou, Guangdong 510535 (CN); ZHANG, Tengxun, Guangzhou, Guangdong 510535 (CN); KUANG, Qianwen, Guangzhou, Guangdong 510535 (CN); TAI, Lihong, Guangzhou, Guangdong 510535 (CN)
(74) Representative: Boult Wade Tennant LLP
(86) International application number: PCT/CN2021/106579
(87) International publication number: WO 2022/213507

(57) **Abstract**

Provided are *Christensenellaceae,* and a composition and a pharmaceutical use thereof. The *Christensenellaceae* can be used for treating or preventing liver function damage and related diseases, digestive tract mucosa damage and related diseases, diabetes, obesity, and related diseases. The *Christensenellaceae* can also be used in combination with hypoglycemic and lipid-lowering drugs, and has a synergistic effect on liver function damage and related diseases, diabetes, and obesity and related diseases.

## Description

The present application claims the priority of Chinese patent applications with application numbers of 202110369840.5 (application date: April 6, 2021, and invention title: " Bacterial strain and composition and use") and 202110370249.1 (application date: April 6, 2021, and invention title: "Drug combinations comprising microorganism and hypoglycemic or lipid-lowering drugs"), respectively, and their entire contents are incorporated in the present application as reference.

### Technical Field

The present application relates to the technical field of bacterial strain isolation and application, in particular, to a bacterial strain and its composition, drug combination and use thereof, the drug combination comprising the bacterial strain and a hypoglycemic or lipid-lowering drug.

### Background

At present, liver disease is mainly prevented and treated by vaccination against hepatitis, reducing alcohol consumption, improving dietary structure, and doing physical exercise. However, these strategies can only play a preventive role, with little benefit, and the effect varies with individual constitutions. Once faced with the liver disease that has occurred, it seems helpless. Therefore, there is an urgent need for countermeasures that can effectively prevent and treat liver diseases, and it is necessary to develop an effective method or drug that can prevent and treat liver diseases with low side effects.

Digestive tract mucosal injury, especially intestinal mucosal injury will not only affect the digestion and absorption of nutrients, but also have extremely adverse effects on the mucosal barrier function and the body's immune function. The repair methods of using the combination of traditional Chinese and Western medicines that commonly used for digestive tract mucosal injury are often accompanied by the disadvantages that the traditional Chinese medicines are slow action, difficult to cure, and easy to relapse, and that the Western medicines are easy to stimulate the digestive system and cause adverse reactions. There is an urgent need to develop drugs with fast action, long-lasting drug effect and no toxic side effects for repairing digestive tract mucosal injury.

Regarding diabetes, there is currently no radical treatment method, and the main methods are to control diabetes through drug therapy. The current drug treatment of diabetes includes oral medication, such as sulfonylurea drugs, biguanide hypoglycemic drugs, α-glucosidase inhibitors, insulin sensitizers, etc., and insulin injection therapy. Although several drugs are available to treat T2D (type 2 diabetes), the efficacy of these drugs varies from person to person, and there are worrisome potential side effects, including: (1) causing gastrointestinal adverse reactions, including nausea, vomiting and diarrhea; (2) increasing islet burden, which may cause pancreatitis; (3) possibly causing goiter and thyroid cancer; (4) other side effects involving intestinal, renal function, hypoglycemia; and, (5) tendency to cause depression. Therefore, there is an urgent need to develop a method or drug for treating diabetes that is effective and has little side effects.

The drug treatment of obesity has a long history, and the currently commonly used weight-loss drugs include liraglutide, orlistat, sibutramine and rimonabant, etc. But many weight-loss drugs are restricted from the market or withdrawn from clinical use because some of them do not work as expected, or because some of them cause serious adverse reactions in patients. Therefore, there is an urgent need to develop an effective method or drug for treating obesity and related diseases thereof with less side effects.

### Summary of the Invention

The objective of the present application is to provide a bacterial strain and its composition, drug combination and use thereof to solve the above technical problems. The drug combination comprises a microorganism and hypoglycemic or lipid-lowering drugs.

Due to the emerging role of intestinal microbes in obesity and diabetes, the use of intestinal microbes to improve diabetes, the interaction between intestinal microbes and antidiabetic drugs, and their impact on drug function have become current research hotspots. On one hand, intestinal microbes can affect the metabolism, immune function and brain function of the host through the secretion of short-chain fatty acids and other pathways, which play an indispensable role in human health. On the other hand, the metabolic activities of intestinal microbes and their metabolites can affect the metabolism and efficacy of drugs, and drugs can also manipulate the composition of intestinal microbes and their metabolic capabilities.

The present application provides a bacterial strain of *Christensenella sp.,* which can treat initial steatohepatitis lesions, slow down liver fat accumulation and relieve liver lesions, thereby effectively preventing liver function damage and related diseases; and the bacterial strain also has the functions of repairing the mucosal barrier of digestive tract, reducing the fasting blood glucose, regulating insulin levels, reducing body weight, and regulating blood lipids, thereby having the functions of preventing and treating digestive tract mucosal injury and related disease, diabetes, obesity and obesity-related disease.

The present application provides a use of a bacterial strain of *Christensenella sp.* species in the manufacture of a medicament for treating or preventing at least one disease or condition selected from at least one of the following: liver function damage and disease related to liver function damage, digestive tract mucosal injury and disease related to digestive tract mucosal injury, diabetes, obesity and obesity-related disease.

The disease related to liver function damage includes at least one of the following diseases: fatty liver, nonalcoholic fatty liver disease, nonalcoholic steatohepatitis, liver fibrosis, liver cirrhosis and liver cancer;

The digestive tract mucosal injury refers to increased permeability of gastrointestinal mucosa, and impaired mucosal barrier function. disease related to digestive tract mucosal injury includes at least one of the following diseases: intestinal leakage, peptic ulcer, gastroenteritis, and inflammatory bowel disease; and

The obesity-related disease includes at least one of the following diseases: obesity, metabolic syndrome, cardiovascular disease, hyperlipidemia, hypercholesterolemia, hypertension, insulin resistance syndrome, obesity-related gastroesophageal reflux disease and steatohepatitis.

In one or more embodiments of the present application, the bacterial strain has a 16s rRNA sequence that is at least 98.65% identical to SEQ ID NO: 1.

In one or more embodiments of the present application, the bacterial strain has a 16s rRNA sequence that is at least 99% identical to SEQ ID NO: 1.

In one or more embodiments of the present application, the bacterial strain has a 16s rRNA sequence that is 99%, 99.5%, 99.9% or 100% identical to SEQ ID NO: 1.

In one or more embodiments of the present application, the above-mentioned medicament is freeze-dried.

In one or more embodiments of the present application, the above-mentioned medicament further comprises one or more pharmaceutically acceptable excipients or carriers.

In one or more embodiments of the present application, the above-mentioned medicament is a vaccine composition.

In one or more embodiments of the present application, the above-mentioned medicament is formulated for oral administration, injection administration or intragastric administration.

In one or more embodiments of the present application, the diabetes includes at least one of the following diseases: type 1 diabetes, type 2 diabetes, insulin resistance syndrome, glucose intolerance, hyperlipidemia, diabetic nephropathy complications, diabetic neuropathy, diabetic eye disease, cardiovascular disease, diabetic foot, and gestational diabetes.

In one or more embodiments of the present application, the dosage forms of the above medicament include tablets, pills, powders, suspensions, gels, emulsions, creams, granules, nanoparticles, capsules, suppositories, injections, sprays and injections.

The present application provides a cell of *Christensenella sp.* strain preserved under the preservation number GDMCC No: 61117 or progeny strain or subclone strain thereof.

The present application provides a composition comprising the above-mentioned *Christensenella sp.* strain and/or metabolites of the strain.

In one or more embodiments of the present application, the above composition further comprises a pharmaceutically acceptable excipient or carrier.

The present application provides a use of a drug combination comprising a microorganism and a hypoglycemic or lipid-lowering drug in the manufacture of a medicament for treating or preventing at least one of the following diseases or symptoms: liver function damage and disease related to liver function damage, diabetes, obesity and obesity-related disease; the microorganism is a bacterium of *Christensenella sp.* species; the hypoglycemic or lipid-lowering drug is one or more drugs capable of improving glucagon-like peptide-1 (i.e., GLP-1) pathway sensitization, supplementing and/or promoting GLP-1 function.

In one or more embodiments of the present application, the above-mentioned hypoglycemic or lipid-lowering drug is at least one of GLP-1 receptor agonist (i.e., GLP-1RA) or GLP-1 mimic, GIP receptor agonist (i.e., glucose-dependent insulin-secreting polypeptide receptor agonist, also known as gastric inhibitory polypeptide receptor agonist), dipeptidyl peptidase-4 (i.e., DPP-4) inhibitor.

The GLP-1 receptor agonist or GLP-1 mimic is at least one selected from the group consisting of exenatide, liraglutide, semaglutide, oral semaglutide, beinaglutide, lixisenatide and exenatide weekly-formulation.

The present application provides a drug combination, which comprises a hypoglycemic or lipid-lowering drug and a microorganism; the microorganism is a bacterium of *Christensenella sp.* species. The hypoglycemic or lipid-lowering drug is one or more drugs capable of improving glucagon-like peptide-1 pathway sensitivity, supplementing and/or promoting GLP-1 function.

The present application provides a use of the above-mentioned composition or the drug combination in the manufacture of a medicament or preparation, and the medicament or preparation is used for at least one of the following:
reducing liver weight;
treating initial steatohepatitis lesions;
slowing down liver cell fat accumulation;
reducing serum AST, ALT;
reducing abdominal white fat inflammatory lesions;
reducing body weight of mammals;
reducing food intake of mammals;
slowing down weight gain rate after drug withdrawal;
reducing body fat in mammals;
reducing the level of at least one of the following indicators in mammals serum: total cholesterol level, low-density lipoprotein and triglyceride level;
increasing the level of high-density lipoprotein in mammals serum;
improving impaired oral glucose tolerance in mammals;
lowering fasting blood glucose in mammals;
reducing HOMA-IR indicator in mammals;
enhancing GLP-1 sensitivity;
enhancing insulin sensitivity;
avoiding GLP-1RA resistance and related side effects caused by gastrointestinal tract dysfunction; and
repairing digestive tract mucosal injury.

The present application provides a composition, which comprises the above-mentioned bacterial strain of *Christensenella sp.* species, or the above-mentioned *Christensenella sp.* strain or progeny strain or subclone strain thereof and/or metabolites thereof, for treating or preventing at least one disease or condition selected from the group consisting of: liver function damage and disease related to liver function damage, digestive tract mucosal injury and disease related to digestive tract mucosal injury, diabetes, obesity and obesity-related disease.

In one or more embodiments of the present application, the aforementioned excipient comprises an antioxidant, a chelating agent, an emulsifier, or a solvent.

The present application provides a drug combination, which comprises a microorganism and a hypoglycemic or lipid-lowering drug, in which the microorganism is the above-mentioned bacterial strain of *Christensenella sp.* species or the above-mentioned *Christensenella sp.* Strain or progeny strain or subclone strain thereof and/or metabolites thereof, the hypoglycemic or lipid-lowering drug is one or more drugs capable of improving glucagon-like peptide-1 pathway sensitivity, supplementing and/or promoting GLP-1 function, and which is used for treating or preventing at least one disease or condition selected from the group consisting of: liver function damage and disease related to liver function damage, diabetes, obesity and obesity-related disease.

The present application provides a method for treating or preventing a disease or condition, comprising administering the above-mentioned composition or the above-mentioned drug combination to a subject in need thereof, in which the disease or condition is at least one selected from the group consisting of: liver function damage and disease related to liver function damage, diabetes, obesity and obesity-related disease.

The present application provides a kit, which comprises the above-mentioned drug combination.

The present application has the following beneficial effects:
The bacterial strain of *Christensenella sp.* provided in the present application can be used for the treatment or prevention of liver function damage and disease related to liver function damage, digestive tract mucosal injury and disease related to digestive tract mucosal injury, diabetes, obesity and obesity-related disease. As verified by the applicant, the bacterial strain of *Christensenella sp.* provided by the present application has no toxic side effects on the kidney and have functions including but not limited to the following: reducing liver weight; treating initial steatohepatitis lesions; slowing down fat accumulation in liver cells; reducing serum AST and ALT; reducing abdominal white fat inflammatory lesions. The bacterial strain of *Christensenella sp.* can also reduce the body's fasting blood glucose, and significantly improve the body's insulin resistance level, and thus has the effect of preventing and treating diabetes. In addition, the bacterial strain of *Christensenella sp.* can also reduce body fat in mammals and improve metabolic function in obese patients. The bacterial strain of *Christensenella sp.* also has the function of repairing damaged digestive tract mucosa and preventing diseases related to mucosal injury.

The drug combination of hypoglycemic or lipid-lowering drug and microorganism provided in the present application can be used for the treatment or prevention of liver function damage and disease related to liver function damage, diabetes, obesity and obesity-related disease. It has been verified by the applicant that the drug combination of GLP-1 receptor agonist or GLP-1 mimic, and the microorganism as provided by the present application has a synergistic technical effect. As compared with the single administration of the microorganism or the single administration of GLP-1 receptor agonists or GLP-1 mimic, the drug combination has better therapeutic effects, the microorganism can enhance the weight loss effect of GLP-1 receptor agonist or GLP-1 mimic, improve abnormal glucose tolerance, and reduce fasting blood glucose. In addition, the drug combination has no toxic and side effects on the kidneys and can reduce liver weight. The drug combination can help to enhance GLP-1 sensitivity; and avoiding GLP-1RA resistance and related side effects caused by intestinal disorders.

### Brief Description of the Drawings

In order to more clearly illustrate the technical solutions of the embodiments of the present application, the attached drawings that are used in the embodiments are briefly described below. It should be understood that the following attached drawings illustrate one some of examples of the present application, and thus should not be regarded as a limitation on the scope, and those skilled in the art can also obtain other related drawings based on these drawings without creative work.
Figure 1 shows the macroscopic morphology diagram of the isolated bacterial strain;
Figure 2 shows the microscopic morphology diagram of the isolated bacterial strain;
Figure 3 shows the macroscopic plate diagram after the anaerobic culture of single bacterium colony;
Figure 4 shows the phylogenetic evolutionary tree;
Figure 5 shows the NASH liver injury scoring standard;
Figure 6 shows the effect of MNO-863 on the liver weight in obese model mice;
Figure 7 shows a diagram of HE staining results of liver tissue;
Figure 8 shows a diagram of oil-red staining results of liver tissue;
Figure 9 shows the NAFLD/NASH liver pathological scores;
Figure 10 shows a diagram of the statistical results of hepatic steatosis degrees;
Figure 11 shows a diagram of the statistical results of hepatic lobular inflammation scores;
Figure 12 shows a diagram of the statistical results of liver ballooning degeneration scores;
Figure 13 shows the levels of AST (aspartate aminotransferase) and ALT (alanine aminotransferase) in serum;
Figure 14 shows the micrograph and the total area statistical chart of white fat inflammatory lesions in the abdomen of obese mice;
Figure 15 shows a diagram of the detection results of blood creatinine (CREA), blood urea (UREA) and blood uric acid (UA) in obese mice;
Figure 16 shows the effect of MNO-863 on oral glucose tolerance in obese mice induced by high-fat diet;
Figure 17 shows the effect of MNO-863 on fasting blood glucose (mmol/L) in obese mice induced by high-fat diet;
Figure 18 shows the effect of MNO-863 on HOMA-IR index in obese mice induced by high-fat diet;
Figure 19 shows the effect of MNO-863 on body weight (g) in obese mice induced by high-fat diet;
Figure 20 shows the effect of MNO-863 on body weight (%) in obese mice induced by high-fat diet;
Figure 21 shows the effect of MNO-863 on the food intake (g) in obese mice induced by high-fat diet;
Figure 22 shows the effect of MNO-863 on TC, TG, LDL, HDL-C in obese mice induced by high-fat diet;
Figure 23 shows the effect of MNO-863 on inguinal fat, subcutaneous fat, and epididymal fat in obese mice induced by high-fat diet;
Figure 24 shows the micrograph of mouse colon tissue of the HFD control group;
Figure 25 shows the micrograph of mouse ileum tissue of the HFD control group;
Figure 26 shows the micrograph of mouse colon tissue of the MNO-863 treatment group;
Figure 27 shows the micrograph of mouse ileum tissue of the MNO-863 treatment group;
Figure 28 shows the micrograph of mouse colon tissue of the NCD control group;
Figure 29 shows the micrograph of mouse ileum tissue of the NCD control group;
Figure 30 shows the effect of MNO-863 in combination with Liraglutide on the absolute body weight of obese mice during the four-week intervention period and the percentage change in body weight;
Figure 31 shows the body weight and the body weight change percentage after four weeks of intervention;
Figure 32 shows the body weight and the body weight change percentage after four weeks of intervention and four weeks of drug withdrawal;
Figure 33 shows the inguinal fat weight after 4 weeks of drug withdrawal to regain weight;
Figure 34 shows the effects of the single administration of MNO-863 strain and the combination administration of the strain and Liraglutide on glucose tolerance in obese mice;
Figure 35 shows the effect of the single administration of MNO-863 strain and the combination administration of the strain and Liraglutide on glucose hyperglycemia in obese mice;
Figure 36 shows the effects of MNO-863 strain on hyperglycemia in obese mice after drug withdrawal for 4 weeks to regain weight;
Figure 37 shows a diagram showing the effect of the single administration of MNO-863 strain and the combination administration of the strain and Liraglutide on liver weight after 4 weeks of drug withdrawal to regain weight.

### Specific Models for Carrying Out the Invention

Reference will now be made in detail to embodiments of the present application, and one or more examples thereof are described below. Each example is provided by way of explanation, not limitation of the disclosure. In fact, it will be apparent to those skilled in the art that various modifications and variations of the present application can be made without departing from the scope or spirit of the present disclosure. For example, the features illustrated or described as part of one embodiment can be used in another embodiment to yield a still further embodiment.

The bacterial strain of *Christensenella sp.* species is used in the manufacture of a medicament for the treatment or prevention of at least one disease or condition selected from the group consisting of liver function damage and disease related to liver function damage, digestive tract mucosal injury and disease related to digestive tract mucosal injury, diabetes, obesity and obesity-related disease.

The drug combination comprising a microorganism and a hypoglycemic or lipid-lowering drug is used in the manufacture of a medicament for the treatment or prevention of at least one disease or condition selected from the group consisting of: liver function damage and disease related to liver function damage, diabetes, obesity and obesity-related disease; the microorganism is a bacterium of *Christensenella sp.;* and/or the hypoglycemic or lipid-lowering drug is one or more drugs capable of improving glucagon-like peptide-1 (GLP-1) pathway sensitivity, complementing and promoting GLP-1 function.

The disease related to liver function damage includes at least one of the following diseases: fatty liver, nonalcoholic fatty liver disease, nonalcoholic steatohepatitis and liver cirrhosis.

In other embodiments, the disease related to liver function damage also includes liver fibrosis and liver cancer.

The digestive tract mucosal injury refers to increased permeability of gastrointestinal mucosa and impaired mucosal barrier function. The disease related to digestive tract mucosal injury includes at least one of the following diseases: intestinal leakage symptom, peptic ulcer, gastroenteritis, inflammatory bowel disease and other diseases; it should be noted that intestinal leakage symptom is manifested by increased intestinal permeability.

The obesity-related disease includes at least one of the following diseases: cardiovascular disease, hyperlipidemia, insulin resistance syndrome, obesity-related gastroesophageal reflux disease, and steatohepatitis.

In other embodiments, the obesity-related disease also includes obesity, metabolic syndrome, hypercholesterolemia, and hypertension.

In other embodiments, the above-mentioned "obesity-related disease" can be selected from the following diseases: overeating, binge eating, hunger, hypertension, diabetes, increased plasma insulin concentration, insulin resistance, hyperlipidemia, metabolic syndrome, insulin resistance syndrome, obesity-related gastroesophageal reflux disease, atherosclerosis, hypercholesterolemia, hyperuricemia, low back pain, cardiac hypertrophy, and left ventricular hypertrophy, lipodystrophy, nonalcoholic steatohepatitis, cardiovascular disease, and polycystic ovary syndrome, and those with obesity-related disorders including those wishing to lose weight.

The three main types of diabetes are type 1 diabetes (T1D), type 2 diabetes (T2D) and gestational diabetes (GDM). Type 1 diabetes is caused by autoimmune damage or idiopathic reasons, is characterized by the absolute destruction of islet function, and mostly occurs in children and adolescents, which must be treated with insulin to obtain satisfactory curative effect, otherwise it will be life-threatening. Type 2 diabetes is a multifactorial syndrome characterized by abnormal carbohydrate/fat metabolism, often including hyperglycemia, hypertension, and abnormal cholesterol. Type 2 diabetes is caused by the inability of insulin to function effectively (less binding to receptor). Therefore, not only fasting blood glucose should be checked, but also 2-hour postprandial blood glucose should be observed, especially islet function test should be performed. There are two types of diabetes during pregnancy: one is diabetes diagnosed before pregnancy, which is called "diabetes combined with pregnancy"; and, the other is diabetes with normal glucose metabolism or underlying impaired glucose tolerance before pregnancy and with onset or diagnosis during pregnancy, which is also known as "gestational diabetes mellitus (GDM)", and more than 80% of pregnant women with diabetes are GDM.

It should be noted that the aforementioned pharmaceutical uses for diabetes include but not limited to the treatment or prevention of type 1 diabetes (T1D), type 2 diabetes (T2D) and gestational diabetes mellitus (GDM).

It should also be noted that the above-mentioned purposes for the treatment of obesity and obesity-related disease includes not only the drug combination of the bacterial strain and the hypoglycemic or lipid-lowering drug provided by the present application, but also other active compounds, and the other active compounds can be a combination of two or more other active compounds. For example, the drug combination is used in combination with an anti-obesity compound, and the anti-obesity compound includes, for example, fenfluramine, dexfenfluramine, phentermine, sibutramine, orlistat, neuropeptide Y5 inhibitor and β3 adrenergic receptor agonist.

In addition, the drug combination is used in combination with a cholesterol-lowering agent, and the cholesterol-lowering agent includes, for example: (i) HMG-CoA reductase inhibitor (lovastatin, simvastatin, pravastatin, fluvastatin, atorvastatin, rivastatin, pitavastatin, rosuvastatin and other statins; (ii) chelate (cholestyramine, colestipol and dialkylaminoalkyl derivative of cross-linked dextran; (iii) nicotinic alcohol, niacin or other salts; (iv) PPARα agonist such as fenofibric acid derivative (gemfibrozil, clofibrate, fenofibrate and bezafibrate); (v) PPARα/γ dual agonist such as KRP-297; (vi) cholesterol absorption inhibitor such as β-sitosterol and ezetimibe; (vii) acetyl CoA, cholesterol acyltransferase inhibitor agent, such as avasimibe; and, (viii) an antioxidant, such as probucol.

In other embodiments, it can also be used in combination with other drugs for inflammation, such as aspirin, non-steroidal anti-inflammatory drug, corticosteroid, sulfasalazine, and cyclooxygenase II selective inhibitor.

In one or more embodiments of the present application, the bacterial strain has a 16s rRNA sequence that is at least 98.65% identical to SEQ ID NO: 1. For example, it has a 16s rRNA sequence that is at least 98.7%, 98.75%, 98.8%, 98.85%, 98.9%, 98.95%, 99%, 99.1%, 99.2%, 99.3%, 99.4%, 99.5%, 99.6%, 99.7%, 99.8%, 99.9% or 100% identical to SEQ ID NO: 1.

In one or more embodiments of the present application, the bacterial strain of *Christensenella sp.* has a 16s rRNA sequence that is at least 99% identical to SEQ ID NO: 1.

In one or more embodiments of the present application, the bacterial strain of *Christensenella sp.* has a 16s rRNA sequence that is 99%, 99.5%, 99.9% or 100% identical to SEQ ID NO: 1.

In one or more embodiments of the present application, the above-mentioned medicament is freeze-dried. Lyophilization is an efficient and convenient technique for preparing a stable composition that allows delivery of the bacterium. The above-mentioned medicament is made into powder or tablets by freeze-drying to facilitate coating or transportation.

In one or more embodiments of the present application, the above-mentioned medicament further comprises one or more pharmaceutically acceptable excipients or carriers.

The pharmaceutically acceptable excipients may be an antioxidant, a chelating agent, an emulsifier, a solvent and the like.

The dosage forms of the medicament include but are not limited to tablets, pills, powders, suspensions, gels, emulsions, creams, granules, nanoparticles, capsules, suppositories, injections, sprays and injections.

The above-mentioned drugs also include pharmaceutically acceptable salts, solvates or stereoisomers thereof, and pharmaceutical compositions formed with one or more pharmaceutically acceptable carriers and/or diluents, which can be prepared in a manner known in the art to formulate into any dosage form that is clinically or pharmaceutically acceptable, and to be administered to patients in need of such treatment by oral administration, injection or intragastric administration. For oral administration, it can be made into conventional solid preparations, such as tablets, capsules, pills, granules, etc.; it can also be made into oral liquid preparations, such as oral solutions, oral suspensions, syrups, etc. When making oral preparations, suitable fillers, binders, disintegrants, lubricants and the like can be added.

In one or more embodiments of the present application, the above-mentioned medicament is a vaccine composition.

In one or more embodiments of the present application, the above-mentioned medicament is formulated for oral administration, injection administration or intragastric administration. Through intragastric administration of mice, the administration of the bacterial strain in the present application exhibits a therapeutic effect equivalent to that of Liraglutide, a drug for treating diabetes.

In other one or more embodiments, the above-mentioned drugs also comprise a pharmaceutically acceptable salt, and the "pharmaceutically acceptable salt" refers to the following salt, which is suitable for contact with tissues of humans and lower animals and does not have undue toxicity, irritation, allergic reaction, etc., and is commensurate with a reasonable benefit/risk ratio.

A cell of the *Christensenella sp.* is preserved under the preservation number GDMCC No: 61117 or progeny strain or subclone strain thereof.

The *Christensenella sp.* MNO-863 provided by the present application was isolated from a stool sample of a healthy male volunteer of Han nationality in Guangzhou City, Guangdong Province. It was deposited in the Guangdong Microbiology Collection Center on August 4, 2020. The preservation number is: GDMCC No: 61117; the preservation address is: Guangdong Institute of Microbiology, 5th Floor, building 59, No. 100 Xianlie Middle Road, Guangzhou City, the test result is survival, and its taxonomic name is *Christensenella sp.*

Macroscopic morphology: the anaerobic culture was performed at 37°C for 72 hours, the colony was light yellow, round, moist surface, translucent, with neat edges. The bacteria were short rod-shaped, without spores, without flagella, immobile, 0.3-0.4 µm × 0.6-1.1 µm, arranged singly or in pairs, Gram-negative. Colony characteristics: MNO-863 was cultured anaerobically at 37°C for 72 hours on a 104 plate, and a single colony was slightly convex, transparent, white, and smooth, and the diameter of the colony was about 0.46-0.50mm.

The present application also provides a composition comprising the above-mentioned bacterial strain of *Christensenella sp.* and/or metabolites of the strain.

The above-mentioned bacterial strain of *Christensenella sp.* can be directly cultured from the above-mentioned strain preserved with the preservation number GDMCC No: 61117, or can be a descendant strain (offspring) or cultured from the original strain (subclone strain), for example, detached cells.

It should be noted that the bacterial strain of *Christensenella sp.* provided in the present application also comprises derivative thereof, for example, it can be modified at the genetic level without eliminating its biological activity. The above-mentioned derivative strain has therapeutic activity and has activity equivalent to that of the bacterial strain of *Christensenella sp.* preserved under the preservation number of GDMCC No: 61117.

In one or more embodiments of the present application, the above composition further comprises a pharmaceutically acceptable excipient or carrier.

A use of the above-mentioned composition in the manufacture of a medicament or preparation, and the medicament or preparation is used for at least one of the following purposes:
reducing liver weight; treating initial steatohepatitis lesions; slowing down fat accumulation in liver cells; reducing serum AST (aspartate aminotransferase) and ALT (alanine aminotransferase); reducing abdominal white fat inflammatory lesions; reducing mammals body weight; reducing mammals food intake; reducing body fat of mammals; reducing the level of at least one of the following indicators in mammals serum: total cholesterol level, low-density lipoprotein and triglyceride levels; increasing the level of mammals serum high-density lipoprotein; improving mammals impaired oral glucose tolerance; reducing fasting blood glucose in mammals; reducing HOMA-IR indicator in mammals; and repairing digestive tract mucosal injury.

The following are purposes in the treatment of liver damage and related diseases: reducing liver weight; treating initial steatohepatitis lesions; slowing down fat accumulation in liver cells; reducing serum AST (aspartate aminotransferase) and ALT (alanine aminotransferase) levels; and, reducing abdominal inflammatory lesions of white fat.

The following are purposes in the treatment or prevention of obesity and related diseases: reducing body weight of mammals; reducing food intake of mammals; reducing body fat of mammals; reducing level of at least one of the following indicators in mammals serum: total cholesterol level, low-density lipoprotein and triglyceride levels; increasing serum high-density lipoprotein level in mammals.

The following are applications or uses in the treatment or prevention of diabetes: improving oral impaired glucose tolerance in mammals, reducing fasting blood glucose in mammals and reducing HOMA-IR indicators in mammals.

The aforementioned repairing of digestive tract mucosal injury refers to repairing digestive tract mucosal injury, typically repairing intestinal mucosal injury. Repairing intestinal mucosal injury refers to achieving at least one of the following indicators: restoring the structural integrity of intestinal mucosal tissue, reducing the atrophy degree of intestinal villi and the number of hyphae.

A drug combination is provided, which comprises a microorganism and a hypoglycemic or lipid-lowering drug; the microorganism is a bacterial strain of *Christensenella sp.* The hypoglycemic or lipid-lowering drug is one or more drugs capable of improving glucagon-like peptide-1 pathway sensitivity, supplementing and/or promoting GLP-1 function.

The above-mentioned hypoglycemic or lipid-lowering drug is GLP-1 receptor agonist (i.e., GLP-1RA) or GLP-1 mimic, GIP receptor agonist (i.e., glucose-dependent insulin-secreting polypeptide receptor agonist, also known as gastric inhibitory peptide receptor agonist), dipeptidyl peptidase-4 (i.e., DPP-4) inhibitor. The GLP-1 receptor agonist or GLP-1 mimic is at least one selected from the group consisting of exenatide, liraglutide, semaglutide, oral dosage form of semaglutide, benaglutide, lixisenatide and exenatide weekly preparation.

The above-mentioned drug combination is used in the manufacture of a medicament or preparation, and the medicament or preparation is used for at least one of the following purposes:
reducing liver weight;
improving oral impaired glucose tolerance in mammals;
lowering fasting blood glucose in mammals;
reducing body weight of mammals; reducing food intake of mammals;
slowing down weight gain rate after drug withdrawal to regain weight;
reducing the level of at least one of the following indicators in mammals serum: total cholesterol level, low-density lipoprotein cholesterol level and triglyceride level;
enhancing GLP-1 sensitivity; avoiding GLP-1RA resistance and related side effects caused by intestinal disorder.

Intestinal flora disorder can easily lead to ineffectiveness and resistance to GLP-1 drugs, while the present inventors propose that the combination of microecologics and GLP-1 can enhance GLP-1 sensitivity and avoid GLP-1RA resistance and related side effects caused by intestinal disorder.

In order to make the purposes, technical solutions and advantages of the examples of the present application clearer, the technical solutions in the examples of the present application will be clearly and completely described below. For those without giving specific conditions in the examples, they are carried out according to the conventional conditions or the conditions recommended by the manufacturer. For those reagents or instruments used without being indicated by the manufacturer, they were all conventional products that could be purchased from the market.

The features and performances of the present application will be described in further detail below in conjunction with the examples.

### Example 1

This example provides the isolation and identification of *Christensenella sp.* MNO-863.

### (1) Isolation of MNO-863

The bacterial strain *Christensenella sp.* MNO-863 of the present application was isolated from a stool sample of a healthy male volunteer of Han nationality in Guangzhou City, Guangdong Province. The volunteer had not used antibiotics in the previous three months when the sample was collected.

Normal saline was dispensed into sterile 10ml centrifuge tubes in a biological safety cabinet; anaerobic blood plate (Jiangmen Kailin anaerobic blood agar medium, Guangdong Mechanical Registration Number: 20172400940) and sterile normal saline were transferred into an anaerobic workbench 24 hours in advance, and 5-7 sterile glass beads were poured into the solidified anaerobic blood plate.

Fresh stool samples at appropriate amount were taken from volunteers and placed in sample preservation tubes containing sterile preservation solution (3% PEG solution, that was, 30g of polyethylene glycol 3350 were weighed and dissolved in 1000mL of normal saline, and sterilized under high pressure at 121°C for 15min), mixed well with a vortex shaker for 10 minutes, then 1mL of solution was taken in the anaerobic workbench, diluted with sterile normal saline to a dilution degree of 10⁻⁶, 0.1mL of the diluted bacterial solution was taken and spread on anaerobic blood agar plate, and incubated in the anaerobic workbench at 37°C for 72 hours. Using the partition and lineation method, single colonies of different shapes were picked with a sterile toothpick and placed on the anaerobic blood agar plate for lineation and isolation culture. After 72 hours of cultivation, colonies with good separation effect were extracted on the partitioned plates for subculture.

### (2) Identification of MNO-863

### ① Microbiological characteristics of MNO-863:

MNO-863 was coated on a solid plate and cultured in liquid to observe microbiological characteristics. The strain coated plate and the liquid culture solution used 104 medium. The formula of 1 L of the medium was shown in Table 1 below:

**Table 1: Formula table of 104 medium.**

| Reagent name | Weighed amount per L of culture medium |
|---|---|
| Pancreatized casein peptone | 5g |
| Peptone | 5g |
| Yeast extract | 10g |
| Beef extract | 5g |
| Glucose | 5g |
| K₂HPO₄ | 2g |
| Tween-80 | 1g |
| Cysteine-HCl×H₂O | 0.5g |
| Sodium acetate | 2g |
| Mother solution of 5× salt solution | 8mL |
| Mother solution of 20× CaCl₂ solution | 2mL |
| Mother solution of 5× chlorhematin | 2mL |
| Mother solution of vitamin K₁ | 0.2mL |

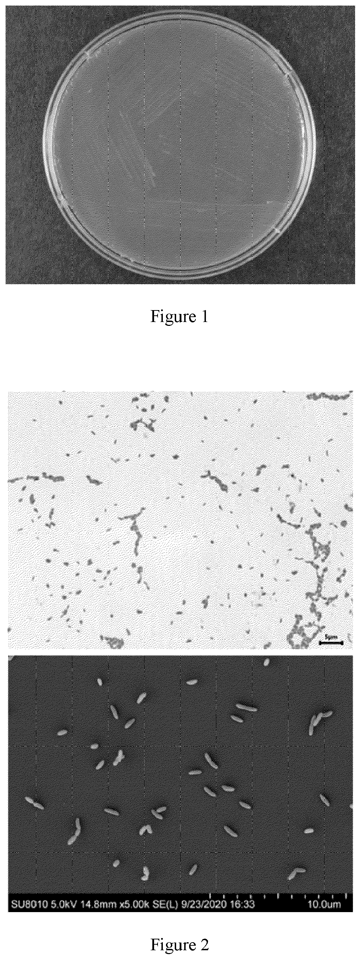

Morphological characteristics: Referring to the macroscopic morphology shown in Figure 1, after anaerobic culture at 37°C for 72 hours, the colonies were light yellow, round, moist, translucent, and had neat edges.

Microscopic morphology: MNO-863 was cultured anaerobically at 37°C for 72 hours on a plate of 104 medium. Gram staining (upper panel in Figure 2) and microscopic examination of spore staining (lower panel in Figure 2) were performed on MNO-863. As shown in Figure 2, the bacteria were short rod-shaped, without spores, without flagella, immobile, 0.3-0.4 µm × 0.6-1.1 µm, arranged singly or in pairs, and Gram-negative.

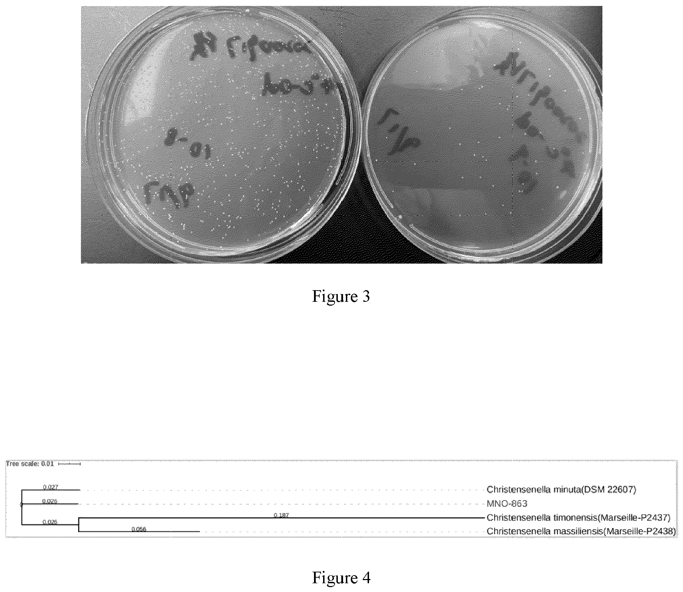

Colony characteristics: MNO-863 was cultured anaerobically at 37°C for 72 hours on a plate of 104 medium, and a single colony was slightly convex, transparent, white, and smooth, and the diameter of colony was about 0.46-0.50mm (see, Figure 3).

The physiological and biochemical characterization of the isolated strains continued. MNO-863 did not grow under aerobic conditions, but grew well under anaerobic conditions, and the optimum growth temperature was 37°C. API 20A reaction kit was used to determine the substrate utilization of MNO-863 and the standard strain *Christensenella minuta* (DSM 22607).

The test results were shown in Table 2. It could be seen from Table 2 that the physiological and biochemical characteristics of the isolated MNO-863 were basically consistent with those of the standard strain DSM 22607, and there was difference when the substrate was glycerol, gelatin hydrolysis, mannose, mannitol and salicyl alcohol.

**Table 2: Comparison of substrate utilization between MNO-863 and standard strain DSM 22607.**

| API 20A substrate | MNO-863 | *Christensenella minuta* (DSM 22607) |
|---|---|---|
| Production of indole | - | - |
| Lactose | - | - |
| Xylose | + | + |
| Glycerol | + | - |
| Melitriose | - | - |
| Urease | - | - |
| Saccharose | - | - |
| Arabinose | + | + |
| Cellobiose | - | - |
| Sorbitol | + | + |
| Glucose | + | + |
| Maltose | - | - |
| Gelatin hydrolysis | + | - |
| Mannose | + | +w |
| Rhamnose | + | + |
| Mannitol | + | +w |
| Salicyl alcohol | +w | + |
| Esculin hydrolysis | - | - |
| Melezitose | - | - |
| Trehalose | - | - |

Explanation of symbols in the above table: "+", positive; "+w", weakly positive; "-", negative.

Cellular fatty acid analysis: The composition and content of phospholipid fatty acids in the cultured MNO-863 and the standard strain *Christensenella minuta* (DSM 22607) were compared and analyzed by gas chromatography. The comparative analysis results were shown in Table 3. It could be seen from Table 3 that, compared with the standard strain, the cellular fatty acid composition of MNO-863 isolated in the present application was significantly different.

**Table 3: Table of cellular fatty acid analysis results.**

| Fatty acid | MNO-863 | DSM22607 |
|---|---|---|
| C9:0 FAME | 0.48 | 0 |
| C11:0 ISO FAME | 27.50 | 2.89 |
| C11:0 FAME | 0.33 | 1.31 |
| C10:0 2OH FAME | 1.33 | 8.57 |
| C12:0 FAME | 4.58 | 1.13 |
| C13:0 ISO FAME | 1.58 | 0 |
| C13:0 ANTEISO FAME | 3.30 | 2.35 |
| C14:0 FAME | 25.01 | 13.03 |
| C15:0 ISO FAME | 20.10 | 27.40 |
| C15:0 ANTEISO FAME | 1.53 | 3.19 |
| C15:0 ISO DMA | 0.60 | 0 |
| C16:0 FAME | 9.34 | 21.14 |
| C17:0 ISO FAME | 0.74 | 1.68 |
| C18:0 FAME | 2.48 | 3.73 |
| C19:0 CYC 9,10 DMA | 1.11 | 0 |

### ② Nucleic acid analysis and identification

### 16srRNA sequencing:

The sequence of the MNO-863 strain was determined by sequencing 16S sequence fragment (the amplification primer and the sequencing primer were 27F: 5'-AGAGTTTGATCCTGGCTCAG-3' and 1492R: 5'-GGTTACCTTGTTACGACTT-3'), and the 16s rRNA sequencing results were shown in the sequence SEQ ID NO: 1:

1 AGTCGAACGA AGTTGCTCTT TGTGAAGCCC TCGGGTGGAA
CTGCGAGTAT

51 ACTTAGTGGC GGACGGGTGA GTAACGCGTG AGCAATCTGC
CCTGCAATGG

101 GGGACAACAG TTGGAAACGA CTGCTAATAC CGCATGAGAC
CACGAAACCG

151 CATGGTTTTG AGGTAAAAGG ATTTATTCGA TGCAGGATGA
GCTCGCGTCC

201 CATTAGATAG TTGGTGAGGT AACGGCCCAC CAAGTCAACG
ATGGGTAGCC

251 GACCTGAGAG GGTGATCGGC CACACTGGAA CTGAGACACG
GTCCAGACTC

301 CTACGGAGGC AGCAGTGGGG AATATTGGGC AATGGGGGAA
AACCCTGACC

351 CAGCAACGCC GCGTGAGGGA AGAAGGTCTT CGGATTGTAA
ACCTTTGTCC

401 TATGGGACGA AACAAATGAC GGTACCATAG GAGGAAGCTC
CGGCTAACTA

451 CGTGCCAGCA GCCGCGGTAA TACGTAGGGA GCAAGCGTTG
TCCGGAATTA

501 CTGGGCGTAA AGGGTGCGTA GGTGGCTATG TAAGTCAGAT
GTGAAAGACC

551 GGGGCTTAAC CCCGGGGTTG CATTTGAAAC TGTGTGGCTT
GAGTACAGGA

601 GAGGGAAGTG GAATTCCTAG TGTAGCGGTG AAATGCGTAG
ATATTAGGAG

651 GAACACCAGT GGCGAAGGCG ACTTTCTGGA CTGTAACTGA
CACTGAAGCA

701 CGAAAGCGTG GGGAGCAAAC AGGATTAGAT ACCCTGGTAG
TCCACGCCGT

751 AAACGATGGA TACTAGGTGT GGGGCCCGAT AGGGTTCCGT
GCCGAAGCTA

801 ACGCATTAAG TATCCCGCCT GGGGAGTACG ATCGCAAGGT
TGAAACTCAA

851 AGGAATTGAC GGGGGCCCGC ACAAGCAGCG GAGCATGTGG
TTTAATTCGA

901 AGCAACGCGA AGAACCTTAC CAAGGCTTGA CATCCTCTGA
CGACTGTAGA

951 GATACAGTTT CCCTTCGGGG CAGAGAGACA GGTGGTGCAT
GGTTGTCGTC

1001 AGCTCGTGTC GTGAGATGTT GGGTTAAGTC CCGCAACGAG
CGCAACCCTT

1051 ATTGCTAGTT GCCAGCGCGT AAAGGCGGGA ACTCTAGTGA
GACTGCCGGG

1101 GACAACTCGG AGGAAGGTGG GGACGACGTC AAATCATCAT
GCCCCTTATG

1151 TCTTGGGCTA CACACGTGCT ACAATGGCCG GTACAAAGGG
CAGCGAACCC

1201 GTAAGGGGAA GCGAATCTCA AAAAGCCGGT CCCAGTTCGG
ATTGTGGGCT

1251 GCAACCCGCC CACATGAAGT CGGAGTTGCT AGTAATCGCG
AATCAGCATG

1301 TCGCGGTGAA TGCGTTCCCG GGCCTTGTAC ACACCGCCCG
TCACACCACG

1351 GAAGTTGGGA GCACCCGAAG CCAGTGGCTT AACCGTAAGG
AGAGAGC

### Evolutionary analysis:

The whole genome of MNO-863 was sequenced, and the MEGA5.0 software was used to display the phylogenetic tree of the 16S rDNA sequence of "MNO-863" and related species by the adjacent junction method. The similarity calculation was repeated 1000 times, and the results were subjected to comparison with genome sequences of standard strains of the Christensenellaceae family in NCBI. The phylogenetic tree showed (see, Figure 4) that MNO-863 was on the same branch as three standard strains *Christensenella minuta* (DSM 22607), *Christensenella timonensis* (Marseille-P2437) and *Christensenella massiliensis* (Marseille-P2438), indicating that MNO-863 belonged to a species of the genus *Christensenella sp.*

Based on the above traditional microbial morphology analysis and nucleic acid analysis, as well as the comparison results with standard strains, it could be considered from a taxonomic point of view that MNO-863 was a species belonging to the genus *Christensenella,* named *Christensenella sp.* MNO-863. It was deposited in the Guangdong Microbiology Collection Center on August 4, 2020. The preservation number was: GDMCC No: 61117; the preservation address was: Guangdong Institute of Microbiology, 5th Floor, Building 59, No. 100 Xianlie Middle Road, Guangzhou City, and the test results were survival. The taxonomic name was *Christensenella sp.*

### Example 2

In this example, an in vivo test of MNO-863 in a high-fat diet-induced obese mouse model was carried out to verify its use in the treatment or prevention of liver function damage and related diseases.

### Experimental Materials:

(1) Experimental animals: 32 C57BL/6J male mice (purchased from Jiangsu Jicui Yaokang Biotechnology Co., Ltd.) were purchased, which were normally fed 5 weeks old mice. The mice were kept in the same environment during the growth process, 8 of them were given SPF grade rat and mice maintenance diet (Guangzhou Hancheng Experimental Equipment Co., Ltd.), and the remaining 24 were given D 12492 high-fat diet (Pike Biological), after feeding for about 8-10 weeks, weighed, and the standard body weight of the diet-induced obesity model reached 38.00±2.00 g.
(2) Test strain: MNO-863 was anaerobically cultured with a culture medium that was 104 liquid medium in Example 1, under anaerobic conditions at 37°C for 48h, until the bacterial concentration was about 10¹¹ CFU/mL order of magnitude, it could be used to gavage as the test article in experiment group. The bacterial solution was stored anaerobically at 4°C.
(3) PBS phosphate buffer solution: It was a mixed solution composed of weak acid and salt thereof, weak base and salt thereof, which could offset and reduce the influence of external strong acid or strong base on the pH of the solution to a certain extent, so as to maintain the pH of the solution relatively stable. The formula of PBS phosphate buffered saline solution was shown in Table 4 below:

**Table 4: PBS phosphate buffer solution formula**

| Reagent name | Weighed amount (g) per liter of buffered solution |
|---|---|
| KH₂PO₄ | 0.24 |
| Na₂HPO₄ | 1.44 |
| NaCl | 8.00 |
| KCl | 0.20 |
| Cy steine-HCl | 0.50 |

### Experimental Procedure:

### (1) Experimental grouping

Eight mice were given SPF grade rat and mice maintenance diet and were completely randomly divided into 2 cages, 4 mice/cage, as the first group. 16 mice with body weight of 38.00g±2.00g were selected from 24 obese mice, and divided into 2 groups (as second group and third group), 8 mice per group, 4 mice/cage. The first group was the control group (NCD-control group) fed with normal diets, the second group was the high-fat diet-induced obesity mouse model group (HFD-control group), and the third group was the bacterial treatment group (MNO-863), the second group and the third group were fed with high-fat diet, and the grouping information was shown in Table 5. After the animals were grouped, sham gavage was performed, and the drug administration was performed one week later. The first and second groups were intragastrically administered with the same amount of PBS phosphate buffer solution, and the third group was intervened by intragastric administration of the MNO-863 test strain, and the intervention lasted for 4 weeks. The amount of intragastric bacterial solution was 0.2mL/10g mouse body weight. The body weight, state, food intake and other data of the mice were recorded every 3 days before and after the modeling and intervention. After administration, animals were dissected and tissues were collected. The use of experimental animals was paid with attention to animal welfare, following the principle of "reduction, substitution and optimization", and was approved by the Experimental Animal Ethics Committee. During the experiment, they were supervised and inspected by the Experimental Animal Ethics Committee.

**Table 5: Experimental grouping**

| No. | Group | Test/control articles | Drug concentration | Administration frequency | Animal number | Diet |
|---|---|---|---|---|---|---|
| 1 | NCD-control | PBS | / | Once per day | 8 | Normal diet |
| 2 | HFD-control | PBS | / | Once per day | 8 | D12492 |
| 3 | MNO-863 | MNO-863 | 1×10¹¹ CFU/mL | Once per day | 8 | D12492 |

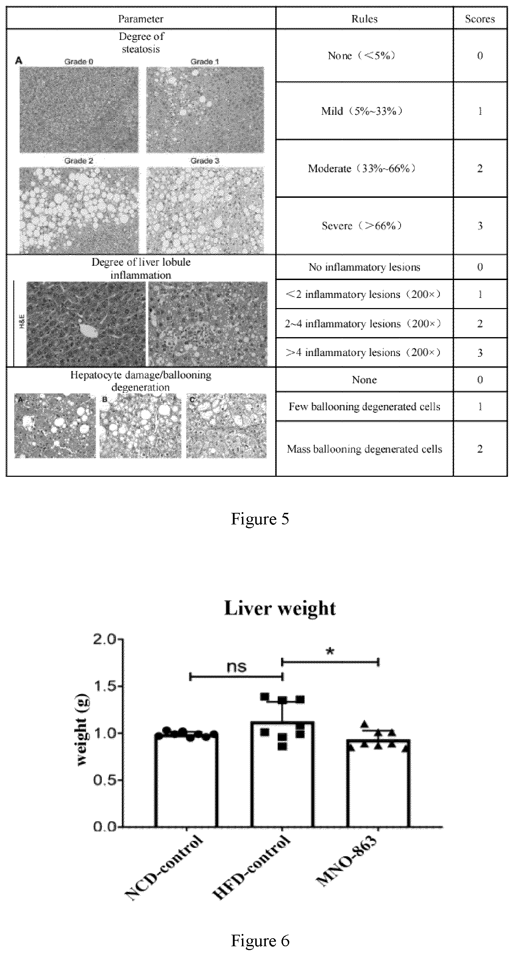

All animals, including those that died and euthanized during the experiment, and sacrificed at the end of the experiment, were subject to gross anatomical examination, and the gross pathological changes of each animal were recorded. The liver was weighed; the middle piece of the large lobe liver was cut and placed in formalin solution, and the remaining tissue was quickly frozen in liquid nitrogen and stored at -80°C. The livers were then made into paraffin-embedded liver pathology sections and stained with HE, Masson, and Oil red. The assessment of liver tissues and NAS pathology interpretation were performed by scoring micrographs captured at the time of original magnification. According to the scoring by the NASH liver injury scoring system (Kleiner DE, Brunt EM, Van NM, Behling C, Contos MJ, Cummings OW, et al. Design and validation of a histological scoring system for nonalcoholic fatty liver disease. Hepatology 2005;41:1313-21.) (Brunt EM. Histopathology of non-alcoholic fatty liver disease. Clin Liver Dis 2009; 13:533-44.), the scoring standard was shown in Figure 5 (0 to 2 points: non-NASH; 3 to 4 points: belonging to unclear; 5 to 8: NASH).

### Experimental results:

The effect of MNO-863 on the liver weight of obese model mice was shown in Figure 6. Compared with the HFD control group, the MNO-863 treatment group could significantly reduce the liver weight of obese mice and returned to the liver weight level of normal NCD control mice. The influence data on the liver weight of obese model mice were shown in Table 6.

**Table 6: Liver weights of mice in different treatment groups**

| Group | Liver weight (g) |
|---|---|
| NCD-control | 0.9875 |
| HFD-control | 1.125 |
| MNO-863 | 0.9325* |

Note: Results were expressed as mean values, *p<0.05, compared with HFD-control group.

(2) Further pathological interpretation of liver lesions in obese model mice: the results showed that MNO-863 could treat initial steatohepatitis lesions, slow down fat accumulation in liver cells, and relieve liver lesions.

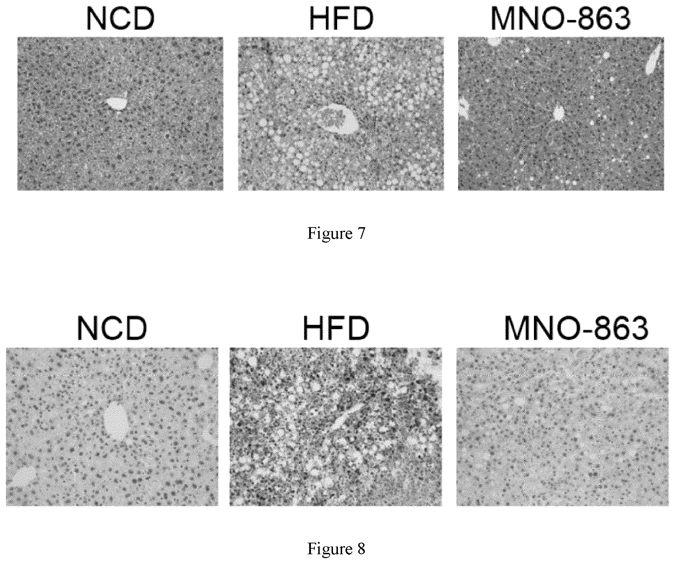

Specifically, HE staining and oil red staining were performed on the mouse liver tissues of the above treatment groups, respectively. The experimental results were shown in Figure 7 and Figure 8, respectively. Under high-fat diet conditions, MNO-863 could treat the initial steatohepatitis lesions and slow down fat accumulation in obese mice.

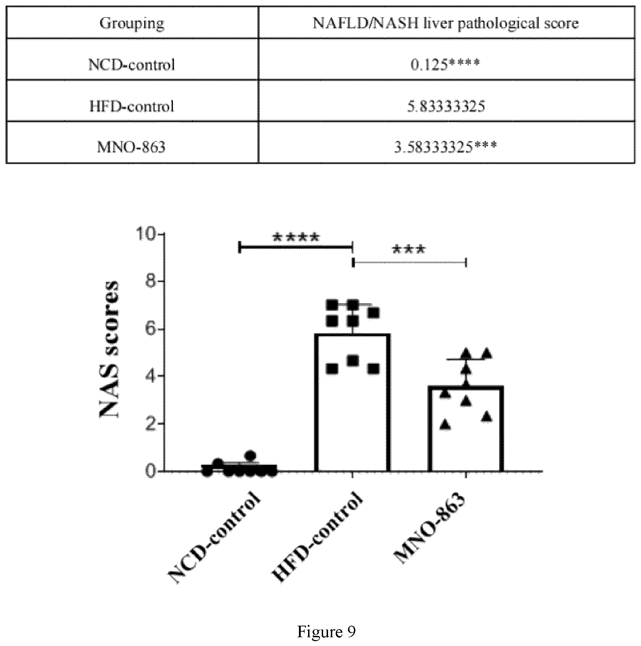

NAFLD/NASH liver pathological scores were shown in Figure 9. According to the scores, MNO-863 could treat the initial steatohepatitis lesions and slow down fat accumulation in obese mice.

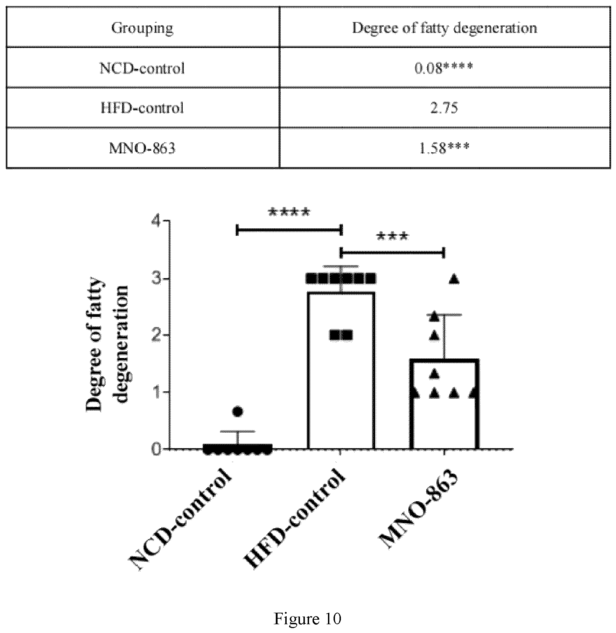

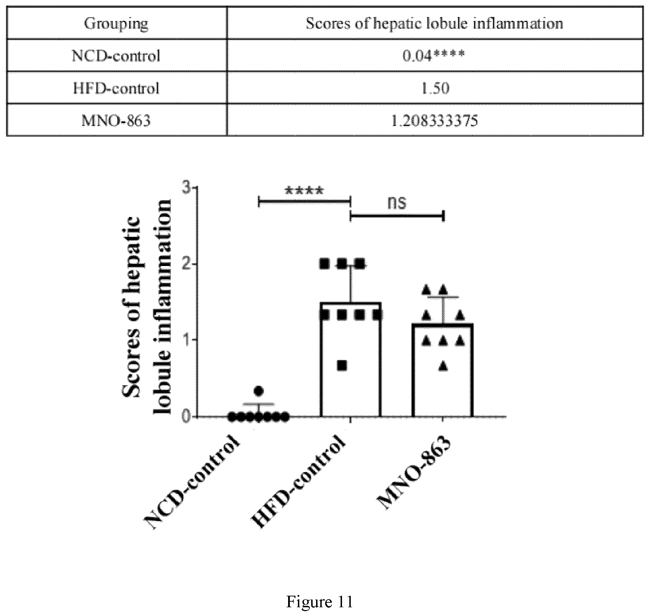

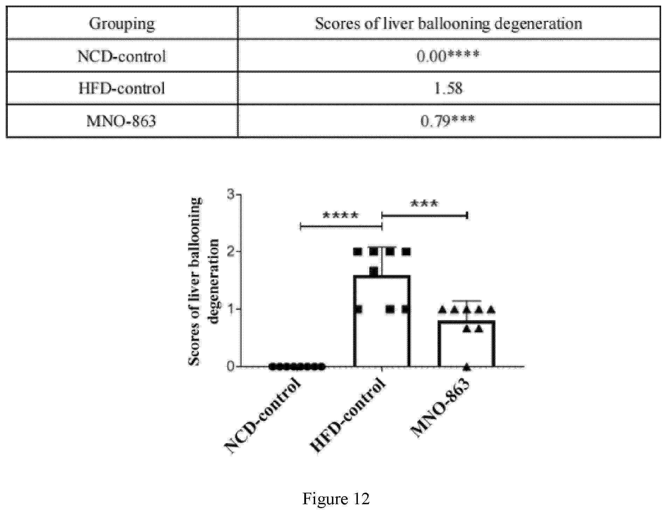

Figure 10 showed the degree of hepatic steatosis, and it could be known from Figure 10 that MNO-863 could effectively alleviate the degeneration of liver fat. Figure 11 showed the score of hepatic lobular inflammation, and it could be seen from Figure 11 that compared with the HFD control group, MNO-863 could effectively inhibit the occurrence of hepatic lobular inflammation. Figure 12 showed the ballooning degeneration score of the liver, and it could be seen from Figure 12 that, compared with the HFD control group, the ballooning degeneration score of the MNO-863 liver decreased significantly.

(3) The applicant also explored the effect of MNO-863 on serum ALT and AST in obese model mice, and the results showed that MNO-863 could significantly reduce serum ALT and AST indicators in obese mice.

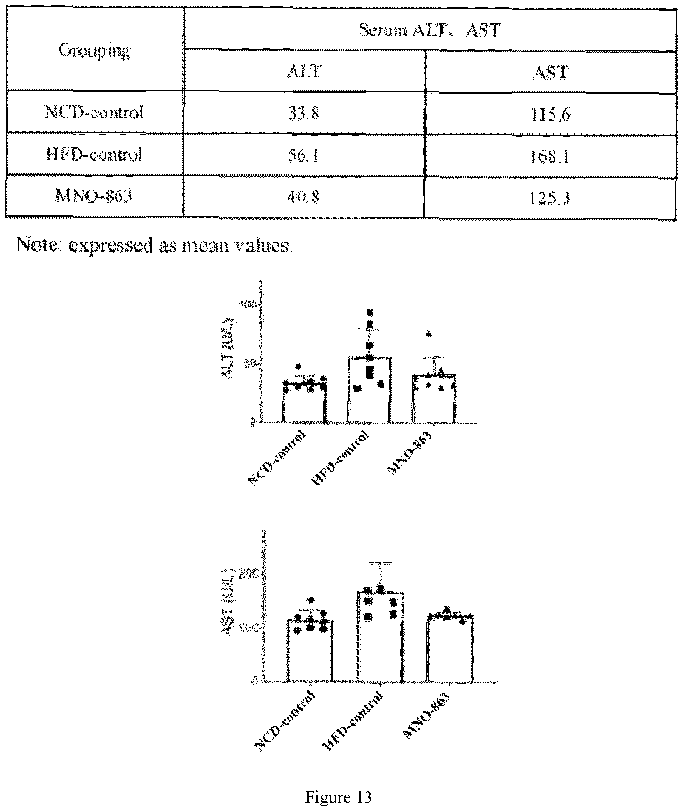

The levels of AST (aspartate aminotransferase) and ALT (alanine aminotransferase) in serum were shown in Figure 13.

(4) The applicant also explored the effect of MNO-863 on the inflammatory lesions of abdominal white fat in obese model mice induced by high-fat diet, and the results showed that MNO-863 could significantly reduce the inflammatory lesions of abdominal white fat in obese model mice.

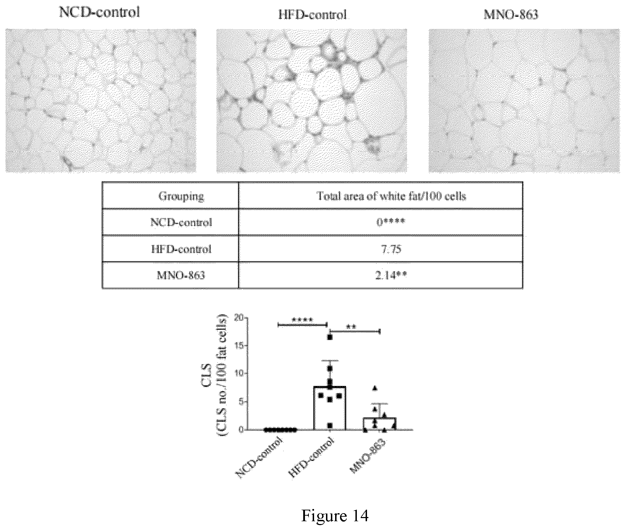

Figure 14 showed the micrographs and total area statistics chart of inflammatory lesions of abdominal white fat of mice.

(5) The applicant also explored the effects of MNO-863 on serum creatinine (CREA), blood urea (UREA) and blood uric acid (UA) in high-fat diet-induced obesity model mice, and the results showed that MNO-863 had no toxicity to the kidneys.

The detection of serum creatinine (CREA) in mice was based on the detection principle of enzymatic method, through the endpoint method (creatinine determination kit, Leishe, S03076), and detected by an automatic biochemical analyzer. The content detection of blood urea (UREA) was based on the detection principle of urease-glutamate dehydrogenase method, and the detection was performed through the two-point method (using urea determination kit, Leishe Life Science Co., Ltd., S03036), and using an automatic biochemical analyzer. The content detection of blood uric acid (UA) was based on the detection principle of uricase method, and the detection was performed through the endpoint method (using uric acid determination kit, Leishe Life Science Co., Ltd., S03035), and using an automatic biochemical analyzer.

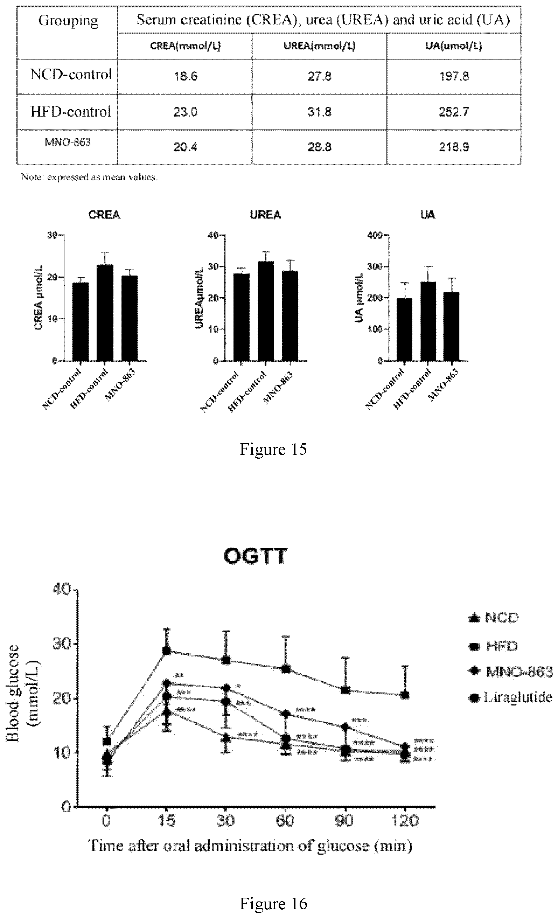

The measurement results of blood creatinine (CREA), blood urea (UREA) and blood uric acid (UA) in mice were shown in Figure 15.

### Example 3

In this example, an in vivo test of MNO-863 in an obese mouse model induced by a high-fat diet was carried out to verify its use in treating or preventing diabetes.

### Experimental Materials:

(1) Experimental animals: 40 C57BL/6J male mice (purchased from Jiangsu Jicui Yaokang Biotechnology Co., Ltd.) were normally fed mice and were 5 weeks old. The mice were kept in the same environment during the growth process. Eight of them were given SPF grade rat and mice maintenance diet (Guangzhou Hancheng Experimental Equipment Co., Ltd.), and 32 were given D12492 high-fat diet (Pike Biological). After feeding for about 8-10 weeks, the mice were weighed, and the body weight of diet-induced obesity model standard reached 38.00±2.00 g.
(2) The strain to be tested: MNO-863 was anaerobically cultured with a medium of 104 liquid medium under anaerobic conditions at 37°C for 48 hours, until the bacterial concentration was on the order of 10¹¹ CFU/mL, it could be used to gavage as the test article in experimental group. The bacteria solution was stored anaerobically at 4°C.
(3) PBS phosphate buffer solution: It was a mixed solution composed of weak acid and salt thereof, weak base and salt thereof, which could offset and reduce the influence of external strong acid or strong base on the pH of the solution to a certain extent, to maintain the pH of the solution relatively stable. The formula of PBS phosphate buffered saline solution was the same as shown in Table 4 of Example 2.
(4) Liraglutide (positive control): Liraglutide was a human glucagon-like peptide-1 (GLP-1) analogue used for the treatment of diabetes, which was purchased from Novo Nordisk, the trade name was Victoza^{®}-Novo Nordisk, and it was injected subcutaneously at 15 µg/kg/day.

### Experimental Procedure:

### (1) Experimental grouping

Eight mice given SPF grade rat and mouse maintenance diet were completely randomly divided into 2 cages, 4 mice/cage, as the first group. 24 mice with body weight of 38.00g±2.00g were selected from 32 obese mice, and divided into 3 groups (as second group, third group, and fourth group, respectively), 8 mice per group, and 4 mice/cage. The first group was the control group fed with normal diet (NCD-control group), the second group was the high-fat diet-induced obesity mouse model group (HFD-control group), and the third group was the microbial treatment group (MNO-863), the fourth group was the liraglutide positive control group, and the second, third and fourth groups were fed with high-fat diet. The grouping information was shown in Table 7. After the animals were grouped, sham gavage was performed, and the drug administration was performed one week later. The first and second groups were intragastrically administered with the same amount of PBS phosphate buffer solution, and the third group was intervened by intragastric administration of the MNO-863 strain to be tested, and the intervention lasted for 4 weeks. The amount of intragastric bacterial solution was 0.2mL/10g mouse body weight. Data such as body weight and state of mice were recorded every 3 days before and after the intervention, respectively. The use of experimental animals was paid with attention to animal welfare, following the principle of "reduction, substitution and optimization", and was approved by the Experimental Animal Ethics Committee. During the experiment, they were supervised and inspected by the Experimental Animal Ethics Committee.

**Table 7: Experimental grouping**

| No. | Group | Test/control articles | Drug concentration | Administration frequency | Animal number | Diet |
|---|---|---|---|---|---|---|
| 1 | NCD-control | PBS | / | Twice per day | 8 | D12450B |
| 2 | HFD-control | PBS | / | Twice per day | 8 | D 12492 |
| 3 | MNO-863 | MNO-863 | 5×10¹¹ CFU/mL | Twice per day | 8 | D 12492 |
| 4 | Liraglutide | Liraglutide | 40µg/mL | Once per day | 8 | D 12492 |

Oral glucose tolerance test (OGTT): On the 28^{th} day after the administration of the animal, the OGTT of fasting for 12 hours was measured (e.g., fasting from 20:30:00 in the evening to 08:30:00 the next day). The mice were weighed for fasting body weight, and intragastrically administrated with glucose according to the fasting weight value of the mice, the dose of intragastrically administrated glucose was 2g/kg (glucose g/mouse fasting weight kg), and the blood-glucose and blood glucose values after glucose administration for 15min, 30min, 60min, 90min, 120min were measured, respectively. Each mouse was strictly timed, and the blood glucose values were measured accurately at 6 time points. Oral glucose tolerance test was a glucose load test, which was used to understand the function of pancreatic β cells and the body's ability to regulate blood glucose, and to observe the patient's ability to tolerate glucose, which was currently recognized as the gold standard for diagnosing diabetes.

After the intervention experiment, the mice were fasted overnight for 10-12 hours, and the fasting body weight was weighed on the next day. Eye blood was collected from the mice after anesthesia with isoflurane (Reward Life Technology Co., Ltd.) by using glucometer (ACCU-CHEK type, Roche) to detect the blood glucose level of fasting blood glucose, and the blood was placed in a refrigerator at 4°C for 3-4 hours. After the blood coagulated and the clot shrunk, centrifugation was performed at 4500r/min for 15min at 4°C, the upper serum was collected, and mouse insulin (INS) enzyme-linked immunoassay kit (Wuhan Huamei Bioengineering Co., Ltd.) was used to detect the content of insulin in serum. HOMA-IR was calculated from fasting-blood glucose and insulin levels in serum. HOMA-IR was an indicator used to evaluate the level of insulin resistance of individuals, and had become a commonly used indicator widely used in clinical evaluation of insulin sensitivity, insulin resistance level and pancreatic β-cell function in diabetic patients, and the calculation method was: fasting blood glucose level (FPG, mmol/L) × fasting insulin level (FINS, µU/mL)/22.5, the HOMA-IR index of normal individuals was 1. With increasing levels of insulin resistance, the HOMA-IR index would be higher than 1. The insulin resistance referred to various reasons that reduced the efficiency of insulin in promoting glucose uptake and utilization, and the body compensatorily secreted excessive insulin to produce hyperinsulinemia to maintain blood glucose stability. Insulin resistance could easily lead to metabolic syndrome and type 2 diabetes.

### Experimental results:

(1) Effect of MNO-863 on oral glucose tolerance of obese model mice: The effect of MNO-863 on oral glucose tolerance of obese mice induced by high-fat diet was shown in Table 8 and Figure 16.

**Table 8: Effect of MNO-863 on oral glucose tolerance in obese mice induced by high-fat diet**

| Group | Blood glucose (mmol/L) | | | | | |
|---|---|---|---|---|---|---|
| | 0 min | 15 min | 30 min | 60 min | 90 min | 120 min |
| NCD | 9.93±2.86 | 17.85±3.66 | 12.95±2.71 | 11.61±1.77 | 10.36±1.68 | 1035±1.86 |
| HFD | 12.11±2.67 | 28.74±3.91 ** | 27.01±5.21 * | 25.44±5.74 **** | 21.54±5.69 *** | 20.66±5.11 **** |
| MNO-863 | 8.3±2.19 | 22.81±6.64 *** | 21.95±6.49 *** | 17.18±6.34 **** | 14.73±3.37 **** | 11.14±2.22 **** |
| Liraglutide | 9.12±1.04 | 20.43±1.33 **** | 19.47±2.26 **** | 12.67±1.37 **** | 10.8±0.92 **** | 9.75±0.43 **** |

| | | | | | | |
|---|---|---|---|---|---|---|
| Note: The results were expressed as mean ± standard deviation (SD), *p<0.05, **p<0.01, ***p<0.001, ****p<0.0001, compared with HFD-control group. | | | | | | |

### When the glucose metabolism was disordered, the blood glucose increased sharply after oral administration of glucose at a certain amount, or increased non-obviously but did not decrease to the fasting level (or the original level) within a short period of time, which was impaired glucose tolerance (IGT) or glucose intolerance. Impaired glucose tolerance (IGT) indicated that the body's ability to metabolize glucose was reduced, and it was common in type 2 diabetes and obesity.

According to the results in Table 8 and Figure 16, after 4 weeks of MNO-863 intervention, the high-fat diet-induced obese mice in the MNO-863 treatment group had significantly lower blood glucose levels than the HFD control group after intragastric administration of glucose for 15 minutes. In the follow-up detection, the blood glucose level of the mice in the MNO-863 treatment group gradually decreased, and after 120 minutes, the blood glucose level returned to close to that of the NCD control group, which was far lower than that of the HFD control group, and there was a significant difference. At the same time, MNO-863 showed therapeutic effects comparable to the diabetes drug liraglutide.

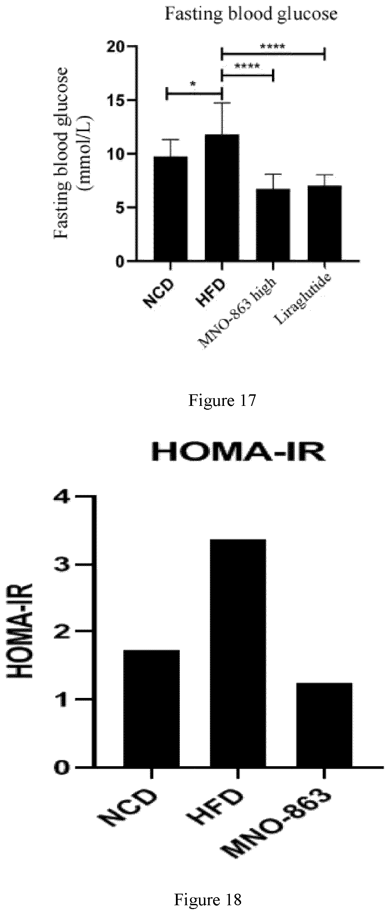

(2) Effect of MNO-863 on fasting blood glucose of obese model mice: The effect of MNO-863 on fasting blood glucose of obese mice induced by high-fat diet was shown in Table 9 and Figure 17.

**Table 9: Effect of MNO-863 on fasting blood glucose (mmol/L) in obese mice induced by high-fat diet**

| Group | Fasting blood glucose (mmol/L) |
|---|---|
| NCD | 9.74±1.53* |
| HFD | 11.81±2.82 |
| MNO-863 | 6.74±1.28**** |
| Liraglutide | 7.02±0.93**** |

| | |
|---|---|
| Note: The results were expressed as mean values, *p<0.05, ****p<0.0001, compared with HFD-control group. | |

According to the results in Table 9 and Figure 17, compared with the HFD control group, the MNO-863 treatment group could significantly reduce the blood glucose level of the high-fat diet-induced obese mice, achieving a significant difference compared with the HFD control group. And compared with the diabetes drug liraglutide, MNO-863 had more obvious control over blood glucose. It showed that MNO-863 had obvious hypoglycemic effect and could improve the symptoms of diabetes.

(3) Effect of MNO-863 on HOMA-IR index of high-fat diet-induced obesity model mice: the effect of MNO-863 on HOMA-IR index of high-fat diet-induced obesity mice were shown in Table 10 and Figure 18.

**Table 10: Effect of MNO-863 on HOMA-IR in obese mice induced by high-fat diet**

| Group | HOMA-IR |
|---|---|
| NCD | 1.74 |
| HFD | 3.36 |
| MNO-863 | 1.24 |

| | |
|---|---|
| Note: The results were expressed as mean values. | |

Insulin resistance (IR) was the main cause of type 2 diabetes, which could promote the occurrence and progression of complications in type 2 diabetes patients. The biochemical indicators related to HOMA-IR could effectively reveal the cause of IR. In general, the HOMA-IR of diabetic patients would be significantly higher than that of normal people.

According to the results in Table 10 and Figure 18, compared with the HFD control group, the intervention of MNO-863 could significantly reduce the HOMA-IR of obese mice induced by high-fat diet. It showed that MNO-863 had the function of improving the body's insulin resistance and pancreatic β cells, so as to achieve the purpose of preventing and treating diabetes.

### Example 4

In this example, an in vivo test of MNO-863 in a high-fat diet-induced obesity mouse model was carried out to verify the application of MNO-863 in the treatment and prevention of obesity and related diseases thereof. Experimental materials (including experimental animals, test strains, PBS phosphate buffer solution and positive control) were the same as those in Example 3.

### Experimental process:

### (1) Test grouping

Eight rats fed with only SPF grade rat and mouse maintenance diet were completely randomly divided into 2 cages, 4 mice/cage, as the first group. 24 mice with body weight of 38.00g±2.00g were selected from 32 obese mice, and divided into 3 groups (as second group, third group, and fourth group, respectively), 8 mice per group and 4 mice/cage. The first group was the control group fed with normal diet (control group), the second group was the high-fat diet-induced obese mouse model group (model group), the third group was the microbial treatment group, and the fourth group was liraglutide-positive control group, the second, third and fourth groups were fed with high-fat diet, and the grouping information was shown in Table 11. After the animals were grouped, sham gavage was performed, then drug administration was carried out one week later. The first and second groups were intragastrically administered the same amount of PBS phosphate buffer solution, and the third group was intervened by intragastric administration of the MNO-863 strain to be tested, and the intervention lasted for 4 weeks. The amount of intragastric bacterial solution was 0.2mL/10g mouse body weight. The body weight, state, food intake and other data of the mice were recorded every 3 days before and after the modeling and intervention. After administration, the animals were dissected and tissues were collected. The use of experimental animals was paid attention to animal welfare, following the principle of "reduction, substitution and optimization", and was approved by the Experimental Animal Ethics Committee. During the experiment, they were supervised and inspected by the Experimental Animal Ethics Committee.

**Table 11: Experimental grouping**

| No. | Group | Test/control articles | Drug concentration | Administration frequency | Animal number | Diet |
|---|---|---|---|---|---|---|
| 1 | NCD-control | PBS | / | Twice per day | 8 | D12450B |
| 2 | HFD-control | PBS | / | Twice per day | 8 | D12492 |
| 3 | MNO-863 | MNO-863 | 5×10¹¹ CFU/mL | Twice per day | 8 | D12492 |
| 4 | Liraglutide | Liraglutide | 40µg/mL | Once per day | 8 | D12492 |

After the experiment, the mice were sacrificed, the fat content was recorded, blood was collected, and the serum was collected by centrifugation at 4500r/min at 4°C for 15min, using assay kit of total cholesterol (TC), triglyceride (TG), high-density lipoprotein (HDL-C) and low-density lipoprotein (LDLC) (Nanjing Jiancheng Bioengineering Institute) to detect the content of blood lipids in serum.

### Experimental results:

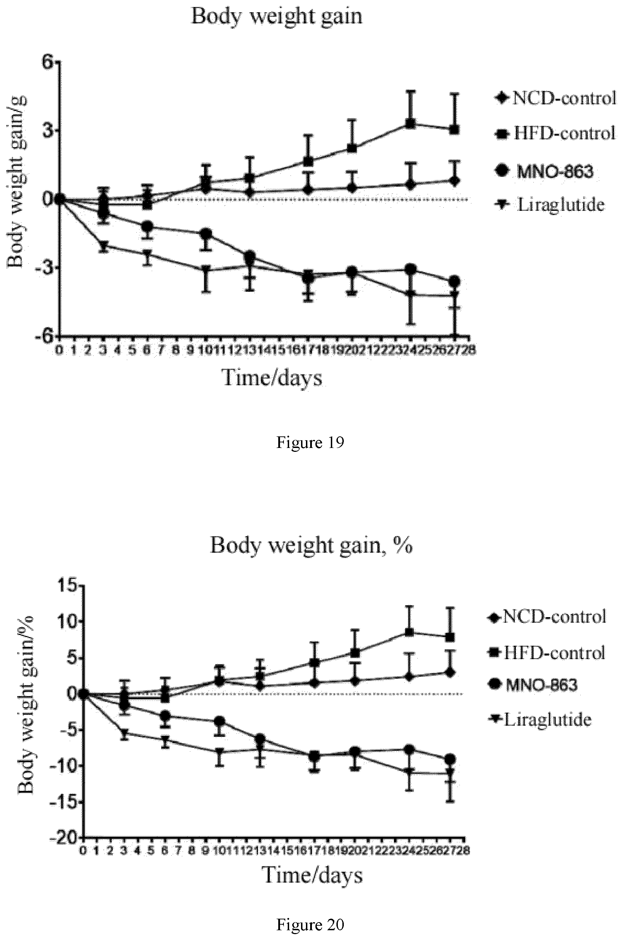

(1) The effect of MNO-863 on the body weight of obese model mice: According to the results in Table 12, Table 13 and Figure 19, Figure 20, compared with the HFD-control group, the MNO-863 intervention group could effectively reduce the body weight of high-fat diet induced-obese mice by more than 3g and the body weight percentage by about 10% within 3 weeks, and reached a significant difference, which was equivalent to the weight loss effect of the positive control drug liraglutide, indicating that MNO-863 had body weight loss effect on the body under high-fat intake.

**Table 12: Effect of MNO-863 on body weight (g) in obese mice induced by high-fat diet**

| Group | Body weight gain (g) | | | |
|---|---|---|---|---|
| | Day 3 | Day 6 | Day 10 | Day 13 |
| NCD | 0.02±0.47 | 0.16±0.45 | 0.47±0.51 | 0.32±0.65 |
| HFD | -0.23±0.57 | -0.24±0.62 | 0.74±0.75 | 0.94±0.89 |
| MNO-863 | -0.6±0.43 | -1.19±0.49 | -1.51±0.67**** | -2.49±0.89**** |
| Liraglutide | -2.03±0.24* * * * | -2.42±0.44**** | -3.12±0.86**** | -2.93±0.95**** |

**Table 13: Effect of MNO-863 on body weight (g) in obese mice induced by high-fat diet**

| Group | Body weight gain (g) | | | |
|---|---|---|---|---|
| | Day 17 | Day 20 | Day 24 | Day 27 |
| NCD | 0.43±0.72 | 0.51±0.67**** | 0.66±0.89**** | 0.83±0.80**** |
| HFD | 1.68±1.09 | 2.22±1.20 | 3.31±1.37 | 3.07±1.51 |
| MNO-863 | -3.46±0.63**** | -3.2±0.80**** | -3.07±1.03**** | -3.61±1.08**** |
| Liraglutide | -3.28±1.06**** | -3.22±0.88**** | -4.2±1.15**** | -4.23±1.55**** |

| | | | | |
|---|---|---|---|---|
| Note: The results were expressed as mean ± standard deviation, ****p<0.0001, compared with HFD-control group. | | | | |

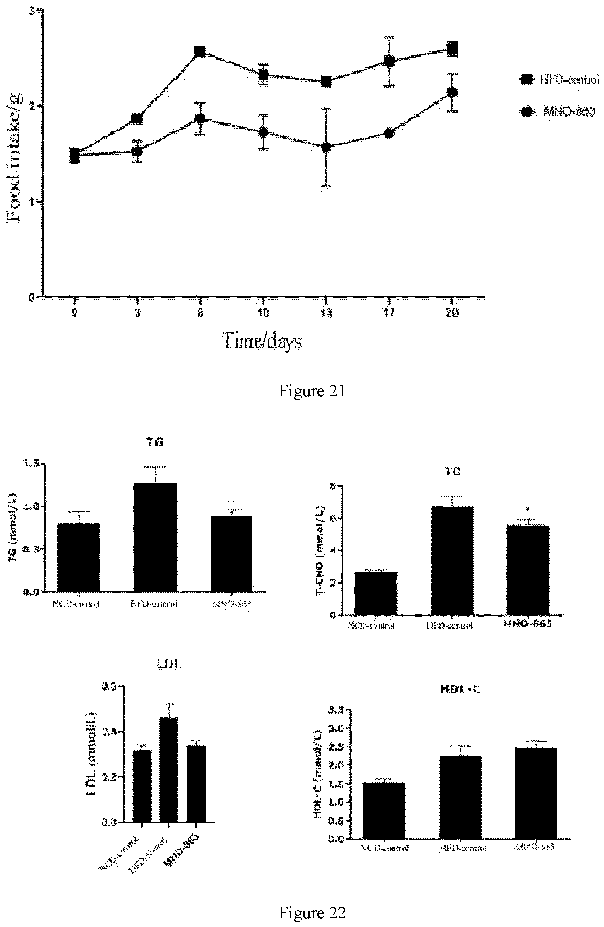

(2) Effect of MNO-863 on food intake of obese model mice: According to the results in Table 14 and Figure 21, compared with the HFD-control group, the MNO-863 intervention group could reduce food intake in obese mice induced by high-fat diet within 3 weeks.

**Table 14: Effect of MNO-863 on food intake (g) in obese mice induced by high-fat diet**

| Group | Food intake (g) | | | | | | |
|---|---|---|---|---|---|---|---|
| | Day 0 | Day 3 | Day 6 | Day 10 | Day 13 | Day 17 | Day 20 |
| HFD | 1.49 | 1.87 | 2.57 | 2.33 | 2.26 | 2.47 | 2.60 |
| MNO-863 | 1.48 | 1.53 | 1.87 | 1.73 | 1.57 | 1.72 | 2.14 |

| | | | | | | | |
|---|---|---|---|---|---|---|---|
| Note: The results were expressed as mean values. | | | | | | | |

(3) Effect of MNO-863 on blood lipids in high-fat diet-induced obesity model mice: According to the results in Table 15 and Figure 22, the MNO-863 intervention group had a significant effect on controlling blood lipid levels of mice under continuous high-fat intake, and could reduce the indicators related to cardiovascular diseases such as primary hyperlipidemia: total cholesterol (TC), triglyceride (TG) and low-density lipoprotein (LDLC), and could elevate blood high-density lipoprotein (HDL-C) level, while HDL was negatively correlated with the incidence and severity of cardiovascular disease, among which the total cholesterol (TC) and triglyceride (TG) results showed significant differences.

**Table 15: Effect of MNO-863 on four items of blood lipids in obese mice induced by high-fat diet**

| Group | Four items of blood lipids | | | |
|---|---|---|---|---|
| | TG | TC | LDL | HDL-C |
| NCD-control | 0.81 | 2.66 | 0.32 | 1.53 |
| HFD-control | 1.27 | 6.76 | 0.46 | 2.26 |
| MNO-863 | 0.88** | 5.57* | 0.34 | 2.47 |

| | | | | |
|---|---|---|---|---|
| Note: The results were expressed as mean values, *p<0.05, **p<0.01, compared with HFD-control group. | | | | |

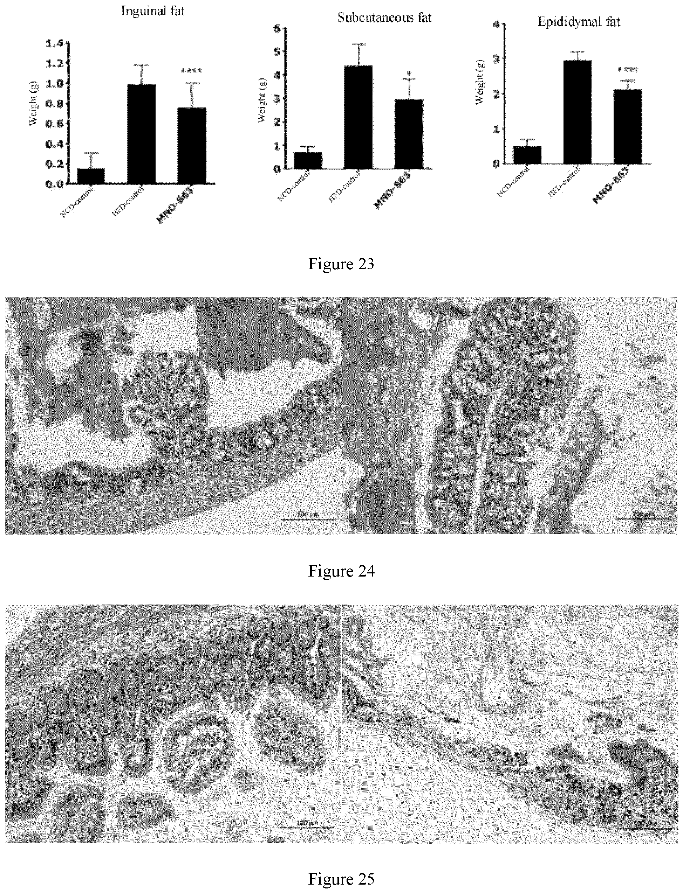

(4) Effect of MNO-863 bacteria on body fat in high-fat diet-induced obesity model mice: According to the results in Table 16 and Figure 23, compared with the HFD-control group, the intervention of MNO-863 significantly reduced the inguinal fat weight, subcutaneous fat weight and epididymal fat weight in obese mice induced by high-fat diet, indicating that MNO-863 had the effect in reducing body fat in mammals.

**Table 16: Effect of MNO-863 on body fat (g) in obese mice induced by high-fat diet**

| Group | Body fat (g) | | |
|---|---|---|---|
| | Inguinal fat | Subcutaneous fat | Epididymal fat |
| NCD-control | 0.15 | 0.73 | 0.50 |
| HFD-control | 1.05 | 4.41 | 2.95 |
| MNO-863 | 0.76**** | 2.99* | 2.12**** |

| | | | |
|---|---|---|---|
| Note: The results were expressed as Mean, *p<0.05, **p<0.01, ****p<0.0001, compared with HFD-control group. | | | |

### Experimental Example 5

In this example, an in vivo test of MNO-863 on mucosal repair of ileum and colon tissues in mouse model was carried out to verify the application of MNO-863 in repairing digestive tract mucosal injury and preventing disease related to digestive tract mucosal injury.

### Animal experiment process:

Eight mice fed with only SPF grade rat and mouse maintenance diet were completely randomly divided into 2 cages, 4 mice/cage, as the first group. 16 mice with body weight of 38.00g±2.00g were selected from 24 obese mice and divided into 2 groups (as second group and third group), 8 mice per group, 4 mice/cage. The first group was the control group fed with normal diet (NCD-control group), the second group was the high-fat diet-induced obesity mouse model group (HFD-control group), and the third group was the microbial treatment group (MNO-863), the second group and the third group were fed with high-fat diet, and the grouping method was the same as Table 5 in Example 2. After the animals were grouped, sham gavage was performed, and the drug administration was started one week later. The first and second groups were intragastrically administered with the same amount of PBS phosphate buffer solution, and the third group was intervened by intragastric administration of the MNO-863 strain to be tested, and the intervention lasted for 4 weeks. The amount of intragastric bacterial solution was 0.2mL/10g mouse body weight. The body weight, state, food intake and other data of the mice were recorded every 3 days before and after the modeling and intervention. After administration, the animals were dissected and the tissues were collected. The use of experimental animals was paid with attention to animal welfare, following the principle of "reduction, substitution and optimization", and was approved by the Experimental Animal Ethics Committee. During the experiment, they were supervised and inspected by the Experimental Animal Ethics Committee.

Dissection and observation process: All animals, including those that died and euthanized during the experiment, and killed at the end of the experiment, were subject to gross anatomical examination. The ileum and colon samples cut from the mice were preserved in formalin solution, sent to Wuhan Saiweier Biotechnology Co., Ltd. to make pathological sections, and photographed for observation.

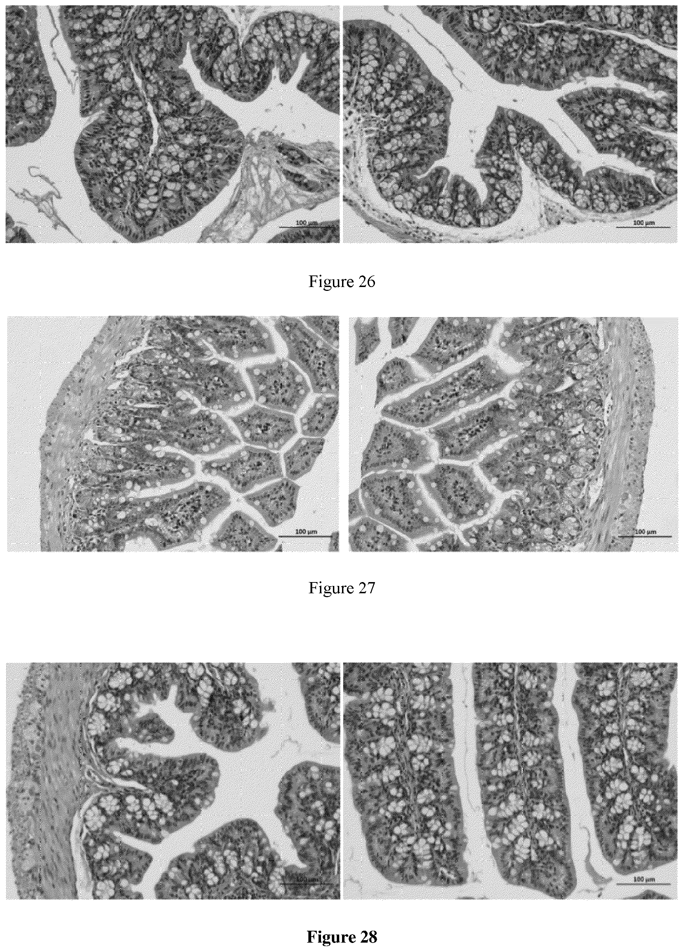

The results showed that the MNO-863 strain to be tested had the function of repairing the mucous layer injury of the mouse ileum and colon tissues (as shown in Table 17): compared with the HFD-control group, under the intervention of MNO-863, the structure of each layer of the mouse colon tissue was clear, the mucosal epithelium was complete, the intestinal glands were abundant and arranged closely, and no obvious abnormalities were observed (as shown in Figure 26). The structure of each layer of the mouse ileum tissue was clear, the intestinal villi were abundant, the mucosal epithelium was complete, the intestinal glands were abundant and arranged closely, and no other obvious abnormalities were observed (as shown in Figure 27). The micrographs of the HFD-control group showed multiple mucosal injuries in the mouse colon tissue, shed mucosal epithelial cells, a small amount of destroyed intestinal gland structure, and appearance of a large number of basophilic hyphae in the intestinal lumen (as shown in Figure 24); damage in mucous membrane layer of mouse ileum tissue, loss of local intestinal villi and mucosal epithelium, disappearance of intestinal gland structure, swilling of a small amount of epithelial cells, loose cytoplasm, and appearance of a large number of basophilic hyphae in intestinal lumen (as shown in Figure 25).

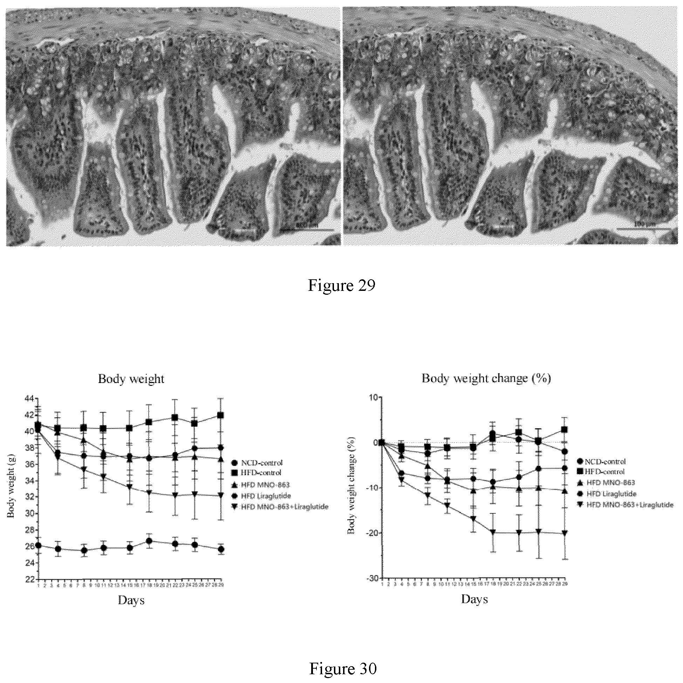

The micrographs of the NCD-control group showed that the structure of each layer of mouse colon tissue was clear, the mucosal epithelium was complete, the intestinal glands were abundant and arranged closely, and no obvious abnormalities were observed (as shown in Figure 28); the structure of each layer of mouse ileum tissue was clear, the intestinal villi were abundant, the mucosal epithelium was complete, the intestinal glands were abundant and closely arranged, and no obvious abnormalities were observed (as shown in Figure 29).

It showed that MNO-863 had the function of repairing the ileum and colonic mucosa, and its application could effectively repair the digestive tract mucosa and had a positive effect on the prevention and treatment of disease related to digestive tract mucosal injury.

**Table 17: Pathological scoring results of MNO-863 on colonic mucosal injury and ileal mucosal injury in mice**

| Pathological scoring results of colon and ileum | | | | |
|---|---|---|---|---|
| Sample No. | Colon | | Ileum | |
| | Mucosal injury | Hypha | Mucosal injury | Hypha |
| HFD-control | 3 | 3 | 2 | 3 |
| NCD-control | 0 | 1 | 0 | 0 |
| MNO-863 | 0 | 0 | 0 | 0 |

The drug combination provided by the present application would be described in further detail below in conjunction with the examples.

In this example, the use of the MNO-863 strain and/or Liraglutide drug was tested in vivo in the high-fat diet-induced obesity mouse model to verify its efficacy in the treatment or prevention of obesity, diabetes and liver diseases.
(1) Experimental animals: 50 C57BL/6J male mice (purchased from Jiangsu Jicui Yaokang Biotechnology Co., Ltd.) were purchased, all of which were raised normally and aged 5 weeks. The mice were kept in the same environment during the growth process, 8 of them were given SPF grade rat and mouse maintenance diet (purchased from Guangzhou Hancheng Experimental Equipment Co., Ltd.), and 42 were fed with D12492 high-fat diet (purchased from Parker Bio) for about 8 to 10 weeks, then the weight was weighed, and the modeling standard of the diet-induced obesity model was that the body weight reached 38.00±2.00 g.
(2) Test Strain: MNO-863 was cultured in anaerobic mode using a culture medium of 104 liquid medium (the formula was shown in Table 1 above), cultured under anaerobic conditions at 37°C for 48 hours, until the bacterial concentration was at magnitude order of about 10⁹ CFU/mL, it could be used to gavage as the test article in the experimental group. The bacterial solution was stored anaerobically at 4°C.
(3) PBS phosphate buffer solution: It was a mixed solution composed of weak acid and salt thereof, weak base and salt thereof, which could offset and reduce the influence of external strong acid or strong base on the pH of the solution to a certain extent, so as to maintain the pH of the solution relatively stable. The formulation of the PBS phosphate buffered saline solution was shown in Table 4 above.

### Experimental process was as follows:

### (1) Experimental grouping was as follows:

Among 42 obese mice, 32 mice with body weight of 38.00g±2.00g were selected and divided into 4 groups, 8 mice per group, 4 mice per cage. The first group was the control group fed with normal diet (NCD control group), the second group was the high-fat diet-induced obesity mouse model group (HFD control group), the third group was the MNO-863 bacterial agent treatment group, the fourth group was the Liraglutide drug treatment group (Lira group), and the fifth group was the treatment group (Lira+MNO-863) using the combination of MNO-863 bacterial agent and Liraglutide drug. Wherein, the second, third, fourth and fifth groups were fed with high-fat diet, and the groups were shown in Table 18. After the animals were grouped, sham gavage was performed, the drug administration was started one week later, and the intervention lasted for 4 weeks. The amount of intragastric bacterial solution was 0.2mL/10g mouse body weight.

The body weight, state, food intake and other data of the mice were recorded every 3 days before and after the modeling and intervention. The animals were dissected for tissue collection at the end of administration. The use of experimental animals was paid with attention to animal welfare, following the principle of "reduction, substitution and optimization", and was approved by the Experimental Animal Ethics Committee. During the experiment, they were supervised and inspected by the Experimental Animal Ethics Committee.

**Table 18: Experimental grouping**

| No. | Group | Test/control articles | Drug concentration | Administration frequency | Animal number | Diets |
|---|---|---|---|---|---|---|
| 1 | NCD-control | PBS | / | Twice per day | 8 | D12450B |
| 2 | HFD-control | PBS | / | Twice per day | 8 | D12492 |
| 3 | MNO-863 | MNO-863 | 5×10¹¹ CFU/mL | Twice per day | 8 | D12492 |
| 4 | Lira | Liraglutide | 40µg/mL | Once per day | 8 | D12492 |
| 5 | Lira+ MNO-863 | Liraglutide+ MNO-863 | 40µg/mL+ 5×10¹¹ CFU/mL | Once per day Twice per day | 8 | D12492 |

### (2) Effect of combined treatment of Liraglutide drug and MNO-863 bacterial agent on body weight.

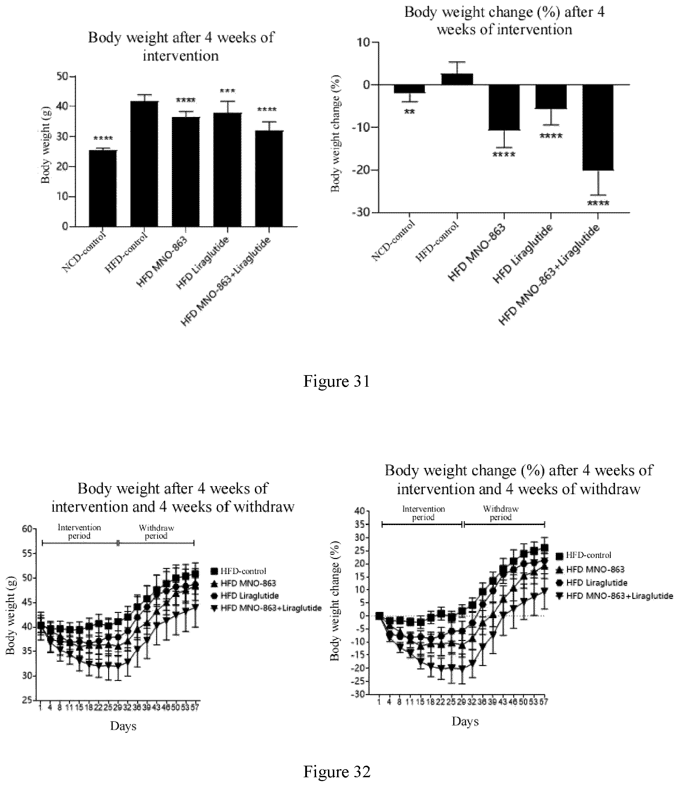

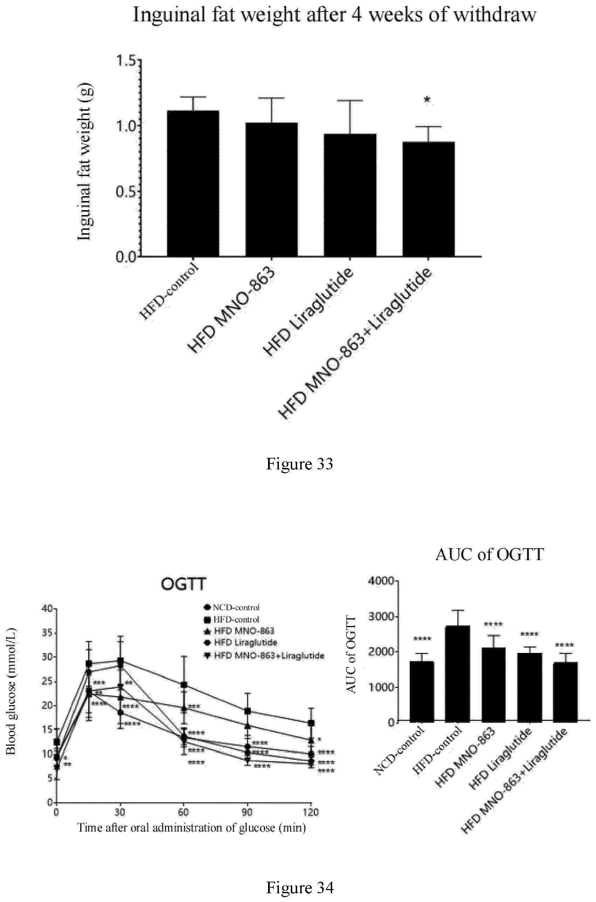

The effect of the combination of MNO-863 and Liraglutide on the absolute body weight of obese mice during the four-week intervention period and the percentage of body weight change were shown in Figure 30, and the body weight and weight change percentage after four weeks of intervention were shown in Figure 31. The body weight and weight change percentage during the four-week intervention period and after four-weeks of drug withdrawal were shown in Figure 32. The inguinal fat weight after 4 weeks of drug withdrawal to regain weight was shown in Figure 33. From Figure 30, Figure 31, Figure 32 and Figure 33, it could be seen that MNO-863 bacterial agent and Liraglutide drug could reduce the body weight of obese mice, inhibit body weight gain thereof, and reduce inguinal fat, while the group using the combination of MNO-863 bacterial agent and Liraglutide drug showed significantly better effects in terms of reducing body weight, inhibiting weight gain and reducing inguinal fat as compared with the single administration of MNO-863 strain or the single administration of Liraglutide drug, indicating that the microecologics could enhance the weight loss effect of drugs.

### (3) Therapeutic effect of combined treatment with Liraglutide and MNO-863 bacterial agent on diabetes.

When the glucose metabolism was disordered, the blood glucose increased sharply after oral administration of a certain amount of glucose, or did not increase obviously, but it did not decrease to the fasting level (or the original level) within a short period of time, which was impaired glucose tolerance (IGT) or glucose intolerance. The impaired glucose tolerance (IGT) indicated that the body's ability to metabolize glucose was reduced, and it was common in type 2 diabetes and obesity.

The therapeutic effect of drugs and bacterial agents on diabetes could be evaluated by glucose metabolism test.

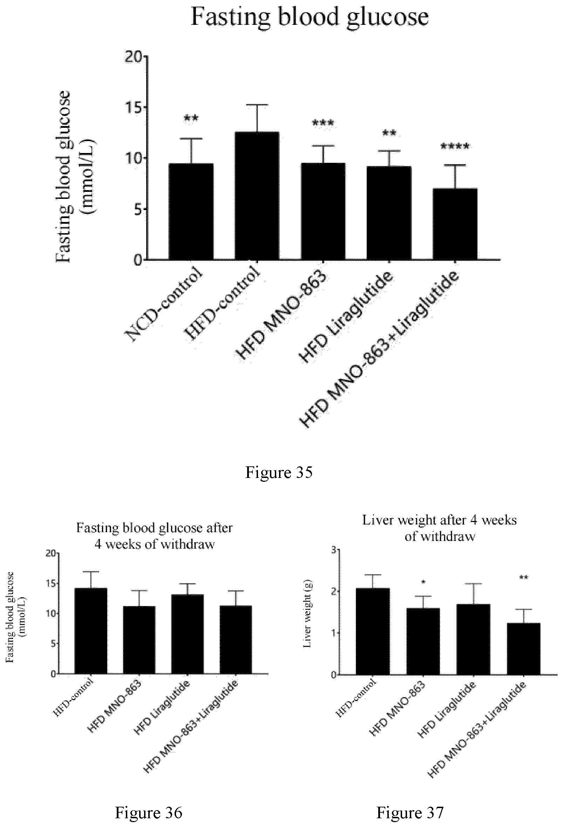

The effects of the single use of MNO-863 strain and the combination use of the strain and Liraglutide on the glucose tolerance in obese mice were shown in Figure 34, the effects of the single use of MNO-863 strain and the combination use of the strain and Liraglutide on the glucose hyperglycemia in obese mice were shown in Figure 35, and the effects of MNO-863 strain on hyperglycemia in obese mice after drug withdrawal for 4 weeks were shown in Figure 36.

From Figure 34, Figure 35 and Figure 36, it could be seen that both MNO-863 bacterial agent and Liraglutide drug could improve abnormal glucose tolerance in obese mice, and could reduce fasting blood glucose, while the group using the combination of bacterial agent and drug showed significant effect compared with the bacterial agent or the drug that was used alone, indicating that the microecologics could enhance the effect of the drug in treating diabetes.

### (4) Therapeutic effect of combination therapy of Liraglutide and MNO-863 bacterial agent on liver diseases.

Figure 37 was a graph showing the effect of the single use of MNO-863 strain and the combination use of the strain and Liraglutide on the liver weight after 4 weeks of drug withdrawal. It could be seen from Figure 37 that both MNO-863 strain agent and Liraglutide could reduce liver weight in obese mice, while the effect of the group using the combination of bacterial agent and drug was more significant than that of the bacterial agent or drug that was used alone, indicating that the microecologics could enhance the effect of drugs in the treatment of liver diseases.

The above descriptions are only typical examples of the present application, and are not intended to limit the present application. For those skilled in the art, the present application may have various modifications and changes. Any of the modifications, equivalent replacements, improvements and so on made within the spirit and principles of the present application shall be included within the protection scope of the present application.

### Industrial Applicability

The bacterial strain of *Christensenella sp.* provided by the present disclosure could be cultivated in large quantities in industry, and the bacterial strain of *Christensenella sp.* provided by the present application could be applied for the treatment or prevention of liver function damage and disease related to liver function damage, digestive tract mucosal injury and disease related to digestive tract mucosal injury, diabetes, obesity and obesity-related disease. The bacterial strain of *Christensenella sp.* provided by the present application has no toxic and side effects on the kidneys and can reduce liver weight; can treat initial fatty liver lesions; can slow down fat accumulation in liver cells; can reduce serum AST and ALT; and can reduce abdominal white fat inflammatory lesions. The bacterial strain of *Christensenella sp.* can also repair the digestive tract mucosa, restore the mucosal barrier function, and prevent intestinal leakage, peptic ulcer and other diseases caused by impaired barrier function. The bacterial strain of *Christensenella sp.* also has the effects of lowering the body's fasting blood glucose, regulating insulin levels, reducing body fat in mammals, preventing and treating diabetes, and improving metabolic functions of obese patients. The bacterial strain of *Christensenella sp.* also has the function of repairing damaged digestive tract mucosa and preventing diseases related to mucosal injury. The drug combination comprising the bacterial strain of *Christensenella sp.* and hypoglycemic or lipid-lowering drug provided by the present application can be produced industrially in large scale, and the drug combination can be used to treat or prevent liver function damage and disease related to liver function damage, diabetes, obesity and obesity-related disease. The drug combination provided by the present application has a synergistic technical effect, that is, the drug combination has a better therapeutic effect than the single administration of the bacterial strain of *Christensenella sp.* or the single administration of hypoglycemic or lipid-lowering drug, and the drug combination has no toxic and side effects on kidneys and can reduce liver weight.

## Claims

1. Use of a bacterial strain of *Christensenella sp.* species in the manufacture of a medicament for treating or preventing at least one disease or condition selected from the group consisting of: liver function damage and disease related to liver function damage, digestive tract mucosal injury and disease related to digestive tract mucosal injury, diabetes, obesity and obesity-related disease.

2. The use according to claim 1, **characterized in that** the disease related to liver function damage comprises at least one of the following diseases: fatty liver, nonalcoholic fatty liver disease, nonalcoholic steatohepatitis and liver cirrhosis;
the digestive tract mucosal injury refers to increased permeability of digestive tract mucosa and impaired mucosal barrier function, and the disease related to digestive tract mucosal injury comprises at least one of the following diseases: intestinal leakage, peptic ulcer, gastroenteritis, and inflammatory bowel disease;
the obesity-related disease comprises at least one of the following diseases: cardiovascular disease, hyperlipidemia, insulin resistance syndrome, obesity-related gastroesophageal reflux disease, and steatohepatitis; and
the diabetes comprises at least one of the following diseases: type 1 diabetes, type 2 diabetes, insulin resistance syndrome, glucose intolerance, hyperlipidemia, complications of diabetic nephropathy, diabetic neuropathy, diabetic eye disease, cardiovascular disease, diabetic foot and gestational diabetes.

3. The use according to claim 1 or 2, **characterized in that**, the bacterial strain has a 16s rRNA sequence that is at least 98.65% identical to SEQ ID NO: 1;
preferably, the bacterial strain has a 16s rRNA sequence that is at least 99% identical to SEQ ID NO: 1; and
preferably, the bacterial strain has a 16s rRNA sequence that is 99%, 99.5%, 99.9% or 100% identical to SEQ ID NO: 1.

4. The use according to any one of claims 1 to 3, wherein the medicament is lyophilized;
preferably, the medicament further comprises one or more pharmaceutically acceptable excipients or carriers; and
preferably, the medicament is a vaccine composition.

5. A cell of the bacterial strain of *Christensenella sp.* preserved under the preservation number GDMCC No: 61117 or progeny strain or subclone strain thereof.

6. A composition, **characterized in that**, it comprises the bacterial strain of *Christensenella sp.* according to claim 5 and/or metabolite thereof; and
preferably, the composition further comprises a pharmaceutically acceptable excipient or carrier.

7. The composition according to claim 6, **characterized in that**, the excipient comprises an antioxidant, a chelating agent, an emulsifier, or a solvent.

8. Use of a drug combination comprising a microorganism and a hypoglycemic or lipid-lowering drug in the manufacture of a medicament for treating or preventing at least one of the following diseases or symptoms: liver function damage and disease related to liver function damage, diabetes, obesity and obesity-related disease; **characterized in that**, the microorganism is a bacterium of *Christensenella sp.* species; the hypoglycemic or lipid-lowering drug is one or more drugs capable of improving glucagon-like peptide-1 pathway sensitivity, supplementing and/or enhancing GLP-1 function.

9. The use according to claim 8, **characterized in that** the hypoglycemic or lipid-lowering drug is at least one of GLP-1 receptor agonist or GLP-1 mimic, GIP receptor agonist, dipeptidyl peptidase-4 inhibitor;
preferably, the GLP-1 receptor agonist or GLP-1 mimic is at least one selected from the group consisting of exenatide, liraglutide, semaglutide, oral dosage form semaglutide, beinaglutide, lixisenatide and exenatide weekly preparation.

10. The use according to claim 8 or 9, **characterized in that**, the bacterium of *Christensenella sp.* has a 16s rRNA sequence that is at least 98.65% identical to SEQ ID NO: 1;
preferably, the bacterium of *Christensenella sp.* has a 16s rRNA sequence that is at least 99% identical to SEQ ID NO: 1; and
preferably, the bacterium of *Christensenella sp.* has a 16s rRNA sequence that is 99%, 99.5%, 99.9% or 100% identical to SEQ ID NO: 1.

11. The use according to any one of claims 8 to 10, **characterized in that** the microorganism is a bacterial strain of *Christensenella sp.* preserved under the preservation number GDMCC No: 61117 or a progeny strain thereof.

12. A drug combination, **characterized in that** it comprises a microorganism and a hypoglycemic or lipid-lowering drug; the microorganism is a bacterium of *Christensenella sp.* species; the hypoglycemic or lipid-lowering drug is one or more drugs capable of improving glucagon-like peptide-1 pathway sensitivity, supplementing and/or enhancing GLP-1 function.

13. Use of the composition according to claim 6 or 7 or the drug combination according to claim 12 in the manufacture of a medicament or preparation, **characterized in that**, the medicament or preparation is used for at least one purpose selected from the following:
reducing liver weight;
treating initial steatohepatitis lesions;
slowing down fat accumulation in liver cells;
reducing serum AST, ALT;
reducing abdominal white fat inflammatory lesions;
reducing body weight of mammals;
reducing food intake of mammals;
slowing down weight gain rate after drug withdrawal;
reducing body fat in mammals;
reducing the level of at least one of the following indicators in mammals serum: total cholesterol level, low-density lipoprotein level and triglyceride level;
increasing the level of serum high-density lipoprotein in mammals;
improving impaired oral glucose tolerance in mammals;
lowering fasting blood glucose in mammals;
reducing mammals HOMA-IR indicators;
enhancing GLP-1 sensitivity;
avoiding GLP-1RA resistance and related side effects caused by intestinal disturbance; and,
repairing digestive tract mucosal injury.

14. A composition, which comprises the bacterial strain of *Christensenella sp.* species according to any one of claims 1 to 4 or the bacterial strain of *Christensenella sp.* according to claim 5 or progeny strain or subclone strain thereof and/or metabolite thereof, and which is used for treating or preventing at least one disease or condition selected from the following: liver function damage and disease related to liver function damage, digestive tract mucosal injury and disease related to digestive tract mucosal injury, diabetes, obesity and obesity-related disease.

15. The composition according to claim 14, **characterized in that** the disease related to liver function damage comprises at least one of the following diseases: fatty liver, non-alcoholic fatty liver disease, non-alcoholic steatohepatitis and liver cirrhosis;
the digestive tract mucosal injury refers to increased permeability of digestive tract mucosa and impaired mucosal barrier function, the disease related to digestive tract mucosal injury comprises at least one of the following diseases: intestinal leakage, peptic ulcer, gastroenteritis, inflammatory bowel disease;
the obesity-related disease comprises at least one of the following diseases: cardiovascular disease, hyperlipidemia, insulin resistance syndrome, obesity-related gastroesophageal reflux disease, and steatohepatitis; and
the diabetes comprises at least one of the following diseases: type 1 diabetes, type 2 diabetes, insulin resistance syndrome, glucose intolerance, hyperlipidemia, complications of diabetic nephropathy, diabetic neuropathy, diabetic eye disease, cardiovascular disease, diabetic foot and gestational diabetes.

16. The composition according to claim 14 or 15, **characterized in that** the bacterial strain has a 16s rRNA sequence that is at least 98.65% identical to SEQ ID NO: 1;
preferably, the bacterial strain has a 16s rRNA sequence that is at least 99% identical to SEQ ID NO: 1; and
preferably, the bacterial strain has a 16s rRNA sequence that is 99%, 99.5%, 99.9% or 100% identical to SEQ ID NO: 1.

17. A method for treating or preventing a disease or condition, comprising administering the composition according to any one of claims 14 to 16 or the composition according to claim 6 or 7 to a subject in need thereof, wherein the disease or condition is at least one selected from the following: liver function damage and disease related to liver function damage, digestive tract mucosal injury and disease related to digestive tract mucosal injury, diabetes, obesity and obesity-related disease.

18. A drug combination, comprising a microorganism and a hypoglycemic or lipid-lowering drug,
the microorganism is the bacterial strain of *Christensenella sp.* species according to any one of claims 1 to 4 or the bacterial strain of *Christensenella sp.* according to claim 5 or progeny strain or subclone strain thereof and/or metabolite thereof,
the hypoglycemic or lipid-lowering drug is one or more drugs capable of improving glucagon-like peptide-1 pathway sensitivity, supplementing and/or promoting GLP-1 function, and used for treating or preventing at least one of the following disease or condition: liver damage and disease related to liver damage, diabetes, obesity and obesity-related disease.

19. A method for treating or preventing a disease or condition, comprising administering the composition according to any one of claims 14 to 16 or the composition according to claim 6 or 7 or the drug combination according to any one of claims 8-11 or the drug combination according to claim 12 or 18 to a subject in need thereof, in which the disease or condition is at least one selected from the following: liver function damage and disease related to liver function damage, diabetes, obesity and obesity-related disease.

20. A kit, which comprises the drug combination according to claim 12 or 18.
